# FASCICULE DE BREVET EUROPEEN

(11) **EP 1 590 373 B1**
(45) Date de publication et mention de la délivrance du brevet: **17.08.2011**
(21) Numéro de dépôt: 04702733.9
(22) Date de dépôt: 16.01.2004
(51) Int. Cl.: C08B 37/00, A61K 39/09, A61K 39/385

(54) **CONJUGUES OBTENUS PAR AMINATION REDUCTRICE DU POLYSACCHARIDE CAPSULAIRE DU PNEUMOCOQUE DE SEROTYPE 5**
DURCH REDUKTIVE AMINIERUNG VON SEROTYP-5-PNEUMOCOCCUS-KAPSELPOLYSACCHARIDERHALTENE KONJUGATE
CONJUGATES OBTAINED BY REDUCTIVE AMINATION OF SEROTYPE 5 PNEUMOCOCCUS CAPSULAR POLYSACCHARIDE

(30) Priorité: 17.01.2003 FR 0300488
(43) Date de publication de la demande: 02.11.2005
(73) Titulaire: Sanofi Pasteur, 69367 Lyon Cedex 07 (FR)
(72) Inventeur: MISTRETTA, Noelle, F-69210 Saint-Bel (FR); DANVE, Emilie, F-69160 Tassin La Demi-Lune (FR); MOREAU, Monique, F-69007 Lyon (FR)
(74) Mandataire: Hurpin, Christian Marcel
(86) Numéro de dépôt international: PCT/FR2004/000089
(87) Numéro de publication internationale: WO 2004/067574

(56) Documents cités:
- WO-A-00/55210
- WO-A-00/56358
- WO-A-98/51339
- PER-ERIK JANSSON: "Structural studies of the pcapsular polysaccharide from streptococcus pneumoniae type 5" CARBOHYDRATE RESEARCH, vol. 140, 1985, pages 101-110, XP008022774 cité dans la demande

## Description

La présente invention a notamment pour objet une forme aminée particulière du polysaccharide capsulaire du pneumocoque type 5, les conjugués incorporant cette forme ainsi que les procédés de fabrication qui permettent de l'obtenir.

Le pneumocoque (*Streptococcus pneumoniae*) est une bactérie encapsulée Gram-positif, responsable de méningites et de bactériémies. Il entraîne également une grande partie des infections respiratoires telles que bronchites, rhinites et otites à complications chez l'adulte comme chez l'enfant. Les pneumocoques sont divisés en sérotypes selon la structure des polysaccharides qui forment la capsule. Le sérotypage des pneumocoques est réalisée à l'aide d'une batterie d'immun sérums, chaque immunsérum étant spécifique d'un seul type de polysaccharide capsulaire. Plus de 90 sérotypes différents, tous pathogènes pour l'homme, ont été recensés. Les sérotypes 6B, 14, 18C, 19F et 23F sont prévalents chez les jeunes enfants et sont la cause de pneumonies et d'otites. Les sérotypes 1 et 5, sont plus fréquemment rencontrés dans les pays en voie de développement que dans les pays industrialisés.

Des vaccins protégeant contre les principaux sérotypes rencontrés en clinique humaine ont été développés ou sont en cours de développement. Un vaccin comprenant les polysaccharides capsulaires de 23 sérotypes différents responsables de 90 % des infections à pneumocoques (1, 2, 3, 4, 5, 6B, 7F, 8, 9N, 9V, 10A, 11A, 12F, 14, 15B, 17F, 18C, 19A, 19F, 20, 22F, 23F, 33F) est efficace chez l'adulte et l'enfant de plus de deux ans. Par contre, l'enfant de moins de deux ans, du fait de l'immaturité de son système immunitaire, ne répond pas à ce vaccin constitué d'antigènes polysaccharidiques T-indépendants. On a surmonté cet obstacle en développant des vaccins contenant des polysaccharides capsulaires de différents sérotypes de pneumocoques couplés (conjugués) à une ou plusieurs protéines porteuses (WO 98/51339). Ces conjugués induisent le développement d'une immunité humorale protectrice T-dépendante chez le jeune enfant, se traduisant par la production d'anticorps spécifiques dirigés contre les polysaccharides des différents sérotypes utilisés dans ces conjugués.

Les polysaccharides capsulaires des différents sérotypes de pneumocoque sont tous formés de la répétition d'une unité de base (unité répétitive) constituée de plusieurs sucres. A titre d'illustration, on indique que l'unité de base du polysaccharide du pneumocoque type 5 est constituée de 5 hexoses : le glucose, la fucosamine N-acétylée, la pneumosamine N-acétylée (2-acetamido-2,6 deoxytalose), l'acide glucuronique et un sucre appelé Sug (2-acetamido-2,6-deoxyhexose-4 ulose) liés les uns aux autres pour former une structure chimique dont la formule condensée est la suivante :

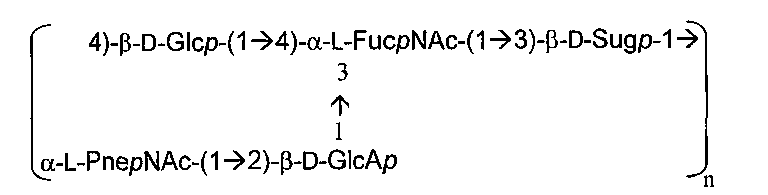

La formule développée qui y correspond est la formule (I) telle que suit : 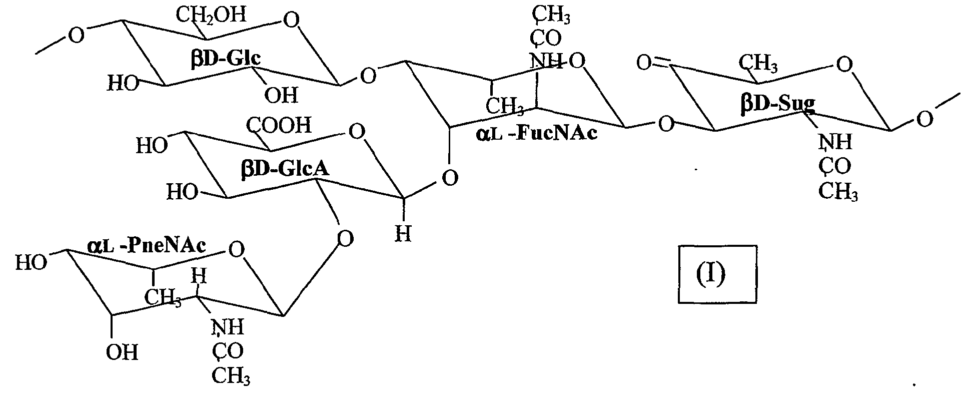 dans laquelle Sug signifie 2-acetamido-2,6-deoxyhexose-4-ulose ; PneNAc signifie 2-acetamido-2,6-deoxytalose, aussi dénommé pneumosamine N-acétylée ; FucNAc signifie 2-acetamido-2,6-deoxygalactose aussi dénommé fucosamine N-acétylée ; GlcA signifie acide glucuronique ; et Glc signifie glucose.

Cette formule a été donnée par Jannson P.E et al., Carbohydrate Research (1985) 140 (1) : 101. Toutefois il n'est pas impossible qu'un faible pourcentage des unités répétitives du polysaccharide présente un groupement hydroxyl à la place de la fonction cétone.

Il existe de nombreux procédés permettant de coupler un polysaccharide à une protéine porteuse. Parmi ceux ci, les procédés qui mettent en jeu à titre préliminaire, une amination réductrice du polysaccharide sont d'usage courant. Par exemple, comme il est décrit dans EP 562 107, le polysaccharide est tout d'abord couplé par amination réductrice à un espaceur bifonctionnel du type NH2-R-NH2 ; puis le polysaccharide ainsi dérivatisé et aminé est couplé à un agent de liaison bifonctionnel notamment capable de réagir avec une fonction amine. Le polysaccharide ainsi activé est ensuite conjugué à une protéine porteuse. Selon d'autres variantes, on peut aussi coupler directement le polysaccharide à la protéine par amination réductrice ou bien encore omettre l'espaceur.

Comme il est bien connu, une réaction d'amination réductrice s'effectue en deux temps. Dans un premier temps, il se forme un composé intermédiaire appelé base de Schiff de formule R-CH=NH+-R', résultant de l'interaction entre un groupement aldéhyde d'une première molécule (R-CHO) et un groupement amine primaire (R'-NH2) d'une deuxième molécule. La base de Schiff est ensuite réduite dans un second temps sous la forme d'un composé aminé R-CH2-NH-R' en présence d'un agent réducteur. On utilise un agent réducteur sélectif capable de réduire la fonction imine de la base de Schiff de manière spécifique, tel qu'un hydrogène activé par un catalyseur, le cyanoborohydrure de sodium (NaCNBH3) ou une amine borane. On utilise de préférence le cyanoborohydrure ou le pyridine borane.

Dans la mesure où tous les polysaccharides possèdent une fonction aldéhyde en bout de chaîne (fonction aldéhyde terminale), les procédés de conjugaison comportant une amination réductrice du polysaccharide sont d'application très générale et lorsqu'il n'existe aucune autre fonction aldéhyde dans l'unité répétitive (fonction aldéhyde intrachaîne), de tels procédés permettent d'obtenir des conjugués dans lesquels une molécule de polysaccharide est couplée à une molécule unique de protéine porteuse.

De manière tout à fait classique, un polysaccharide est soumis à une amination réductrice, en présence d'un agent réducteur sélectif de la base de Schiff, pendant au moins 24 à 48 heures, à un pH neutre ou basique.

Ainsi, US 4,761,283 décrit l'amination réductrice du polysaccharide capsulaire du pneumocoque de type 6A (préalablement fragmenté par hydrolyse acide) par un mutant non toxique de la toxine diphtérique (mutant CRM 197), en présence de cyanoborohydrure de sodium. Le pH du milieu réactionnel est basique (pH=8) et la durée d'incubation est de 18 jours à 37°C.

EP 477 508 décrit l'amination réductrice des polysaccharides du pneumocoque de type 6A, 14, 19F et 23F par du diaminométhane ou du diaminoéthane, en présence de pyridine borane qui joue le rôle d'agent réducteur de la base de Schiff. Le pH du milieu réactionnel est de 9.2. La réaction se poursuit pendant 48 hrs.

Dans EP 562 107 cité plus haut, l'amination réductrice du polysaccharide est conduite à pH 8 pendant 6 jours.

Dans la mesure où le polysaccharide capsulaire du pneumocoque de type 5 comporte une fonction cétone intrachaîne, on devrait en théorie s'attendre à ce que cette fonction cétone, en plus de la fonction aldéhyde terminale, soit amino-réduite au cours de l'amination réductrice du polysaccharide. Or, lorsque l'on soumet en parallèle le polysaccharide capsulaire du pneumocoque de type 5 et un polysaccharide capsulaire d'un autre sérotype ou d'une autre espèce ne comportant pas de fonctions aldéhyde et cétone intrachaînes, les taux d'amination sont équivalents ; ce qui semble bien confirmer que seule la fonction aldéhyde terminale du polysaccharide capsulaire du pneumocoque de type 5 est aminée et que seule cette fonction est modifiée au cours de la réaction. Or, en vérité, tel n'est pas le cas.

En effet, on a maintenant découvert que la structure chimique de l'unité répétitive du polysaccharide du pneumocoque de type 5 était modifiée après amination réductrice selon la méthode classique. Ceci a été montré par spectrométrie de résonance magnétique nucléaire (RMN) et par chromatographie d'échange d'anions haute performance couplée à une détection ampérométrique en champ pulsé (chromatographie HPAEC-PAD) : La grande majorité du résidu Sug disparaît au profit de trois nouveaux composés: (i) la β-D-quinovosamine N-acétylée issue de la réduction de la fonction cétone du résidu Sug en fonction alcool et (ii) un composé X résultant d'une transformation plus importante du résidu Sug. Le troisième composé n'est autre que l'isomère de la β-D-quinovosamine N-acétylée, c'est-à-dire la β-D-fucosamine N-acétylée, issue également de la réduction du résidu Sug et dont on observe une légère augmentation.

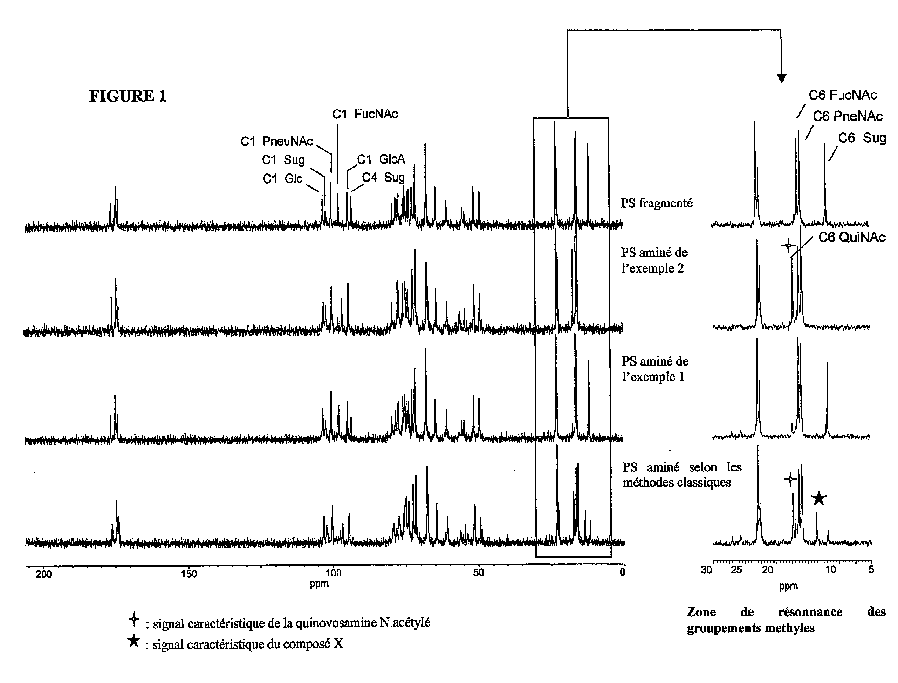

En spectrométrie RMN et tel que montré à la Figure 1, le polysaccharide du pneumocoque de type 5 sous sa forme native simplement fragmentée, présente dans la zone de résonance des carbones des groupements méthyles en position 6 (C6) des sucres, les trois signaux de résonances caractéristiques des groupements méthyles de la pneumosamine N-acétylée (PneNAc), de la fucosamine N-acétylée (FucNAc) et du résidu Sug (Fig. 1-spectre 1). La fragmentation ne modifie en rien le spectre mais en améliore sa résolution. Après amination classique par le diaminohexane, en présence de cyanoborohydrure de sodium (NaCNBH3), on note que le signal de résonance caractéristique du groupement méthyle du résidu Sug (en abrégé « signal caractéristique du résidu Sug ») est substantiellement diminué et que cette diminution s'accompagne de l'apparition de deux nouveaux signaux de résonances : l'un entre 17 et 18 ppm, caractéristique de la quinovosamine N-acétylée (QuiNAc) et l'autre entre 13 et 14 ppm, qui signale un composé nouveau, dont la structure n'est pas connue et qui, pour cette raison, est dénommé composé X (Fig. 1-spectre 4).

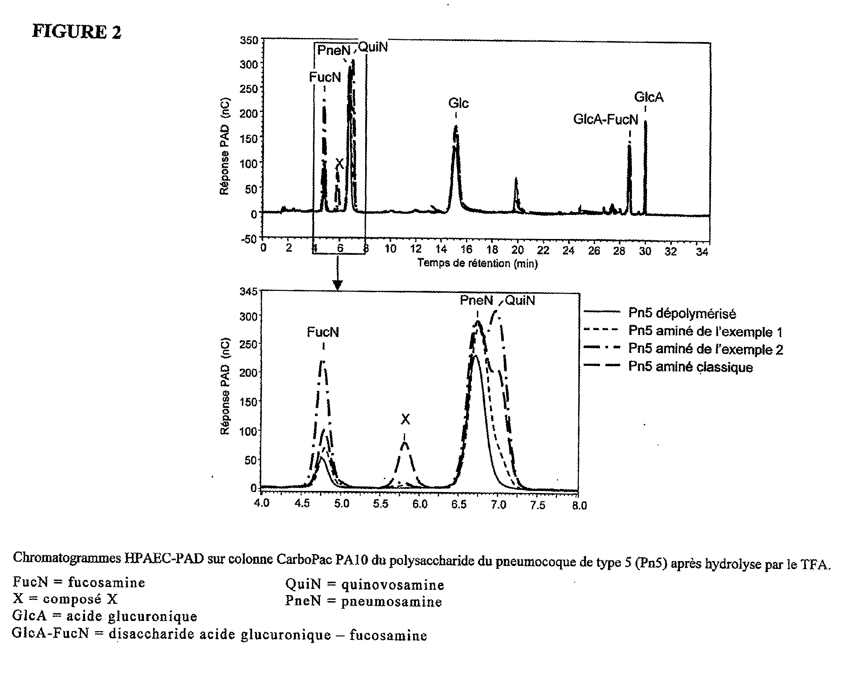

En chromatographie HPAEC-PAD, lorsque l'on compare les chromatogrammes des produits de l'hydrolyse par l'acide trifluoroacétique 2N, pendant 2 heures à 120°C du polysaccharide natif ou dépolymérisé et du polysaccharide obtenu après amination réductrice classique, tels que montré à la Figure 2 (courbe en tiret cadratin), on constate l'apparition d'un premier pic (entre 5,50 et 6,10 min quand la chromatographie est réalisée dans les conditions spécifiées plus loin) correspondant à un composé issu de l'amination réductrice dont la structure est non-identifiée (composé X) ainsi qu'un deuxième pic (entre 6,90 et 7,40 min quand la chromatographie est réalisée dans les conditions spécifiées plus loin) caractéristique de la quinovosamine. D'autre part, l'intensité du pic correspondant à la fucosamine est sensiblement augmentée. Il convient de noter que les chromatogrammes, y compris celui du polysaccharide natif ou dépolymérisé, ne comportent pas de pic correspondant au résidu Sug. En effet, pour être soumis à une chromatographie HPAEC-PAD, le polysaccharide doit tout d'abord être hydrolysé. Cette hydrolyse détruit le résidu Sug ; elle a aussi pour effet de convertir la QuiNAc, le PneNAc et la FucNAc en quinovosamine (QuiN), pneumosamine (PneN) et fucosamine (FucN).

D'autre part, on a également découvert que la transformation du composé Sug en composé X était néfaste à l'immunogénicité du polysaccharide, même si cette transformation n'est que partielle - c'est-à-dire n'ayant lieu que dans certaines des unités répétitives du polysaccharide et non dans la totalité. Par contre, la transformation en QuiNAc ou FucNAc n'a aucune influence notable.

Il est donc apparu souhaitable de rechercher les moyens qui permettaient d'éviter l'apparition du composé X. Ceci a été rendu possible en modifiant le mode opératoire classique de l'amination réductrice. De manière alternative, on peut aussi réduire au préalable les fonctions cétone (C=O) du polysaccharide natif (non-fragmenté). Ceci peut être mis en oeuvre en utilisant un agent réducteur fort tel que le NaBH4. Cet agent réducteur ne génère pas de composé indésirable. Il ne réduit que les fonctions cétone et aldéhyde. Mais comme il réduit ces dernières, il est nécessaire de fragmenter ensuite le polysaccharide réduit afin de réintroduire des groupements aldéhydes terminaux. La réduction des fonctions cétone empêche toute modification ultérieure lorsque le polysaccharide est soumis par la suite à une amination réductrice quelconque.

C'est pourquoi l'invention a pour objet :
(i) Un polysaccharide capsulaire du pneumocoque de type 5 aminé au niveau du groupement aldéhyde terminal et qui présente (i) un spectre RMN du Carbone (¹³C) dépourvu de signal de résonance entre 13 et 14 ppm bornes incluses ; (ii) un chromatogramme HPAEC-PAD, substantiellement dépourvu de pic entre les pics de fucosamine et pneumosamine, le chromatogramme étant obtenu par élution à partir d'une colonne Carbopac^{™} PA10, dans une solution de soude 18 mM, à un débit de 1 ml/min pendant 15 min, des monosaccharides issus de l'hydrolyse dudit polysaccharide ; ou (iii) les deux.
(ii) Un conjugué dans lequel le polysaccharide selon l'invention est couplé à un polypeptide porteur (P).
(iii) Un premier procédé de fabrication d'un polysaccharide capsulaire du pneumocoque de type 5 aminé, selon lequel on soumet le polysaccharide à une amination réductrice en présence d'un agent réducteur sélectif d'une base de Schiff, à un pH de 4 à 6,5, de préférence de 5 à 6, pendant une durée n'excédant pas 4 heures.
(iv) Un deuxième procédé de fabrication d'un polysaccharide capsulaire du pneumocoque de type 5 aminé, selon lequel (i) on fait réagir le polysaccharide avec un agent capable de réduire une fonction cétone, (ii) on fragmente le polysaccharide réduit obtenu en (i), et (iii) on soumet le polysaccharide réduit et fragmenté à une amination réductrice.

Dans la suite du texte, on nomme « polysaccharide aminé » un polysaccharide capsulaire du pneumocoque de type 5 qui est aminé au niveau du groupement aldéhyde terminal ; c'est-à-dire issu de la réaction de la fonction aldéhyde terminale avec une fonction amine.

L'amination réductrice d'un polysaccharide peut être réalisée en faisant réagir la fonction aldéhyde terminale d'un polysaccharide avec des composés très divers mais bien évidemment tous caractérisés en ce qu'ils possèdent au moins une fonction amine primaire. Ces composés peuvent être des polypeptides ou des composés chimiques. Ces polypeptides et ces composés chimiques seront évoqués de manière plus détaillée dans la suite de la description. Lorsqu'il s'agit d'un polypeptide, le produit de l'amination réductrice est en fait un conjugué polysaccharide-polypeptide.

Pour usage dans les procédés selon l'invention, un polysaccharide capsulaire du pneumocoque de type 5 est au préalable avantageusement purifié à partir d'une culture bactérienne par exemple selon la méthode de Gotschlich et al, J. Exp. Med. (1969) 129 : 1349.

Le polysaccharide peut être utilisé tel quel (on parle alors de forme native) ou bien fragmenté. En effet, la taille du polysaccharide n'est absolument pas critique. Sous sa forme native, le polysaccharide contient environ 300 unités répétitives. Un polysaccharide fragmenté peut être constitué d'au moins 4 unités répétitives, en général de 25 à 100 unités répétitives. Si nécessaire, on peut déterminer la taille d'un polysaccharide selon des méthodes connues, par exemple par la détermination de son KD par gel filtration ou par la mesure de sa masse moléculaire par SEC-triple détection (chromatographie d'exclusion-diffusion couplée à une triple détection : réfractométrie, viscosimétrie, diffusion de la lumière).

Un polysaccharide peut être fragmenté selon diverses méthodes bien connues de l'homme du métier, par exemple par hydrolyse acide ou basique ménagée ou dépolymérisation radicalaire oxydative telle que décrite dans EP 562 107.

Dans le premier procédé selon l'invention, on utilise de préférence un polysaccharide fragmenté ; dans le deuxième, un polysaccharide natif.

Afin de caractériser la présente invention, la spectrométrie de résonance magnétique nucléaire (RMN) peut être mise en oeuvre selon des protocoles tout à fait conventionnels ; en particulier les données peuvent être recueillies à partir de tout type d'appareil destiné à cet effet. La spectrométrie RMN du Carbone a déjà été largement utilisée pour étudier les unités répétitives d'un certain nombre de polysaccharides capsulaires, notamment ceux du pneumocoque. A titre d'exemple, on cite C. Jones et al, Carbohyd. Res. (2000) 325 : 192. L'homme du métier dans le domaine de l'analyse spectrale dispose donc de suffisamment d'information pour mettre en oeuvre de lui-même l'analyse du polysaccharide de type 5 par spectrométrie RMN du Carbone.

Néanmoins, à titre d'exemple, on indique un protocole de préparation des échantillons qui est notamment approprié pour mesure ultérieure dans un spectrophotomètre Bruker DRX500 avec une sonde de mesure large bande (largeur spectrale : 27500 Hz). 12 à 17 mg d'un lyophilisat d'un polysaccharide de type 5 sont dissous dans 0.5 ml d'eau deutérée (D2O). Cet échantillon est placé dans un tube de 5 mm spécialement conçu pour l'analyse RMN. Puis les spectres sont enregistrés à 70°C (343 K).

Aux fins de caractérisation, la chromatographie HPAEC-PAD doit s'effectuer dans des conditions bien précises portant sur le type de colonne, les solutions d'élution et le débit d'élution. Ci-après on fournit un mode opératoire complet.

Il convient tout d'abord d'hydrolyser les polysaccharides aminés en monosaccharides. Pour ce faire, on traite 5 à 20 µg de polysaccharide aminé en solution dans de l'eau dé-ionisée à une concentration de 10-40 µg/ml, par 500 µl d'acide trifluoroacétique 2 N pendant 2 heures à 120°C en flacon bouché hermétiquement. Les hydrolysats sont séchés sous un flux d'azote à 40°C de façon à éliminer l'acide trifluoroacétique. Les résidus sont alors dissous dans 400 µl d'eau déionisée.

La chromatographie est mise en oeuvre sur une colonne analytique CarboPac^{™} PA10 (4 x 250mm) commercialisée par Dionex. Cette colonne est constituée d'un support à base de polystyrène et de divinyl benzène sulfonatée ayant un degré de réticulation de 55 % recouvert de microbilles de latex greffées avec des groupements ammonium quaternaire. Le degré de réticulation du latex (microbilles de latex) est de 5 % ; le diamètre des microbilles est de 400 nm.

On injecte 5 µg d'hydrolysat dans la colonne. Puis on soumet la colonne à un flux d'une solution de soude 18 mM pendant 15 min à un débit de 1 ml/min, afin d'éluer les monosaccharides et oligosaccharides non chargés tels que les hexoses et les hexosamines. Pour parachever le chromatogramme on peut ensuite augmenter graduellement la molarité de la solution de soude jusqu'à 100 mM et enfin éluer les monosaccharides et oligosaccharides acides restants à l'aide d'une solution de soude 100 mM/acétate de sodium 300 mM. Pendant toute la durée de l'élution le débit est de 1ml/min et la température est de 30°C.

Dans ces conditions, la distance entre les pics de fucosamine et de pneumosamine est d'environ 2 min. Lorsque les pics de fucosamine et de pneumosamine apparaissent à 4,75 et 6,75 min respectivement, le pic X s'il doit apparaître, sort entre 5,50 et 6,10 (5,80 min).

Les produits obtenus par chacun des deux procédés selon l'invention répondent tous deux à la définition du produit selon l'invention ; mais diffèrent en ce qui concerne le pourcentage de composé Sug et de quinovosamine N-acétylée.

Lorsque le premier procédé selon l'invention (qui spécifie des conditions d'amination réductrice optimisée) est mis en oeuvre, on obtient un polysaccharide aminé qui présente:
(i) Un spectre RMN du Carbone qui comprend un signal de résonance entre 11,5 et 12,5 ppm, bornes incluses, caractéristique du composé Sug, et un signal de résonance situé entre 17 et 18 ppm bornes incluses, caractéristique de la quinovosamine N acétylée, dont l'intensité est moindre par comparaison avec le signal situé entre 17 et 18 ppm, bornes incluses, dans le spectre RMN (¹³C) d'un polysaccharide capsulaire du pneumocoque de type 5 obtenu après amination réductrice, en présence de cyanoborohydrure de sodium, à pH 8, pendant 48 heures ; ou
(ii) Un chromatogramme HPAEC-PAD qui comprend un pic élué immédiatement après la pneumosamine, caractéristique de la quinovosamine dont l'intensité est moindre par comparaison avec le pic équivalent dans le chromatogramme HPAEC-PAD d'un polysaccharide capsulaire du pneumocoque de type 5 obtenu après amination réductrice, en présence de cyanoborohydrure de sodium, à pH 8, pendant 48 heures ; ou
(iii) Les deux.

Il n'est pas possible de quantifier dans l'absolu le pourcentage de résidus Sug qui ont été modifiés après amination réductrice, dans une molécule de polysaccharide prise séparément ou dans un ensemble de molécules. Par contre, il est possible d'indiquer que le taux de modification des résidus Sug est diminué d'au moins 65 % - quand ce n'est pas d'au moins 70 ou 75 % - lorsque le polysaccharide capsulaire du pneumocoque de type 5 est obtenu sous forme aminée par le premier procédé selon l'invention, par comparaison avec un polysaccharide capsulaire du pneumocoque de type 5 aminé selon un procédé classique. Après amination réductrice selon le premier procédé selon l'invention, l'essentiel des modifications qui persistent, porte sur la transformation de certains résidus Sug du polysaccharide en quinovosamine N-acétylée ; le composé X n'est pas formé de manière substantielle comme en témoigne le spectre RMN du Carbone et le chromatogramme HPAEC-PAD (Figure 1 -2^{éme} spectre- et Figure 2 -courbe en tirets-). En effet, le spectre ne présente pas de signal de résonance compris entre 13 et 14 ppm bornes incluses, caractéristique du composé X et le chromatogramme présente simplement un pic large de très faible intensité entre le pic de fucosamine et de pneumosamine (entre 5,50 et 6,10 min).

Par le premier procédé selon l'invention, il est même possible d'obtenir un polysaccharide aminé qui présente (i) un spectre RMN du Carbone carrément dépourvu de signal de résonance entre 17 et 18 ppm ; (ii) un chromatogramme HPAEC-PAD dépourvu de pic quinovosamine (entre 6,90 et 7,40 min), le pic observé avec le polysaccharide aminé selon un procédé d'amination classique s'étant réduit jusqu'à n'être plus qu'un simple épaulement du pic précédent (pic de la pneumosamine) ; ou (iii) les deux.

Le deuxième procédé selon l'invention requiert au préalable la réduction des groupements cétone du polysaccharide natif. Dans ces conditions, on comprend aisément que les résidus Sug soient essentiellement transformés en quinovosamine et fucosamine N-acétylées. Dans ce cas là on parle de « polysaccharide réduit ». L'amination réductrice selon un mode opératoire classique du polysaccharide réduit et fragmenté n'a aucune influence notable sur la structure des unités répétitives. Ceci est bien montré à la Figure 2 -courbe en tiret point-.

Ainsi, lorsque le deuxième procédé selon l'invention est mis en oeuvre, on obtient un polysaccharide aminé qui présente:
(i) Un spectre RMN du Carbone dépourvu de signal de résonance entre 11,5 et 12,5 ppm, bornes incluses caractéristique du composé Sug, qui comprend un signal de résonance situé entre 17 et 18 ppm bornes incluses, caractéristique de la quinovosamine N-acétylée, dont l'intensité est augmentée par comparaison avec le signal de résonance situé entre 17 et 18 ppm, bornes incluses, dans le spectre RMN (¹³C) d'un polysaccharide capsulaire du pneumocoque de type 5 obtenu après amination réductrice, en présence de cyanoborohydrure de sodium, à pH 8, pendant 48 heures ; ou
(ii) Un chromatogramme HPAEC-PAD qui comprend un pic situé immédiatement après le pic de pneumosamine, caractéristique de la quinovosamine, dont l'intensité est augmentée par comparaison avec le pic équivalent dans le chromatogramme HPAEC-PAD d'un polysaccharide capsulaire du pneumocoque de type 5 obtenu après amination réductrice, en présence de cyanoborohydrure de sodium, à pH 8, pendant 48 heures ; ou
(iii) Les deux.

En d'autres termes, un polysaccharide aminé selon l'invention, aminé au niveau du groupement aldéhyde terminal, est essentiellement constitué d'unités répétitives de formule (II) 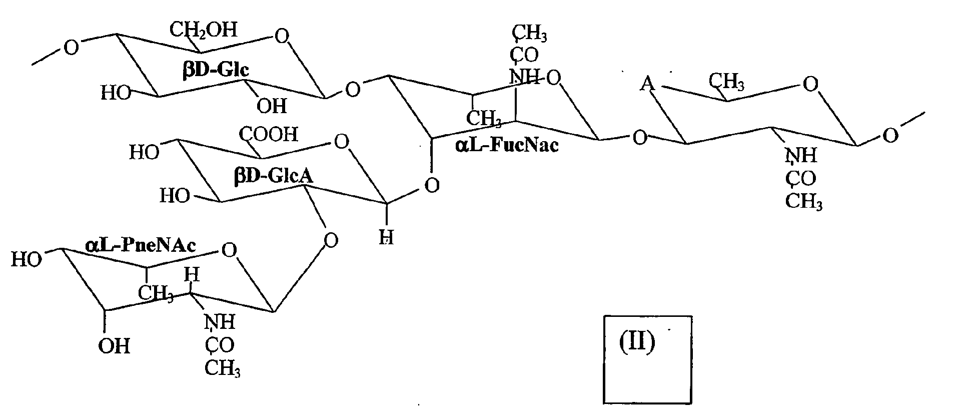 dans laquelle A est de manière indépendante et aléatoire C=O ou CHOH.

Par « polysaccharide aminé essentiellement constitué » on entend un polysaccharide aminé au niveau du groupement adéhyde terminal dans lequel le pourcentage d'unités répétitives de formule (II) est d'au moins 85 %, de préférence d'au moins 90 %, de manière tout à fait préférée d'au moins 95 %.

En fonction du procédé utilisé pour fabriquer des polysaccharides aminés selon l'invention, la proportion des unités répétitives de formule (II) dans laquelle A est C=O (formule (II') peut varier considérablement ; et par voie de conséquence il en est de même de la proportion des unités répétitives de formule (II) dans laquelle A est CHOH (formule (II").

Le deuxième procédé selon l'invention génère un polysaccharide aminé dans lequel les unités répétitives répond à la formule (II") puisqu'en RMN, le signal de résonance correspondant au résidu Sug disparaît au profit du signal de résonance correspondant à la quinovosamine N-acétylé . Le polysaccharide contient au moins 95 % d'unités répétitives de formule (II").

Par contre, en utilisant le premier procédé selon l'invention, on produit un polysaccharide aminé dans lequel le rapport unités de formule (II') / unités de formule (II") est bien différent. En effet, en RMN, le signal de résonance correspondant au résidu Sug est visible ainsi que celui correspondant à la quinovosamine N-acétylée. Le polysaccharide contient de 85 à 95 % d'unités répétitives de formule (II').

Comme signalé précédemment, un polysaccharide selon l'invention peut être aminé à l'aide de divers composés. Lorsque le polysaccharide selon l'invention est destiné à la fabrication de conjugués, le composé permettant d'aminer le polysaccharide est avantageusement choisi selon la structure que l'on veut donner à ces conjugués. De manière générale, les conjugués selon la présente invention répondent à la formule Ps -*CH2-NH-*R dans laquelle :
Ps désigne le polysaccharide capsulaire du pneumocoque type 5 essentiellement constitué des unités répétitives de formule (II) ;
R est un polypeptide porteur P ;
   un composé de formule (III) L-P, dans laquelle un polypeptide porteur est lié à un agent de liaison (L) ; ou
   un composé de formule (III') S-L'-P, dans laquelle un polypeptide porteur P est lié à un espaceur (S) par l'intermédiaire d'un agent de liaison (L') ;
C désigne l'atome de Carbone du groupement aldéhyde terminal du polysaccharide ; et N désigne l'atome d'azote du groupement amine apporté par R.

Les composés P, L et S seront décrits plus loin dans la description, dans la section consacrée aux conjugués. On note cependant dès à présent que ces composés doivent tous bien évidemment comporter un groupement amine primaire libre capable de réagir avec une fonction aldéhyde. Ils peuvent être employés dans chacun des deux procédés selon l'invention, sans distinction aucune.

Dans le premier procédé selon l'invention, l'amination réductrice à laquelle est soumis le polysaccharide consiste notamment à faire réagir le polysaccharide avec un composé comportant une fonction amine tel que les composés P, L ou S, en présence d'un agent réducteur sélectif d'une base de Schiff, tel que le cyanoborohydrure ou le pyridine borane, dans les conditions de pH et de temps déjà spécifiées, de préférence entre 30 min et 4 heures.

Afin d'obtenir un taux d'amination similaire à celui obtenu dans des conditions classiques, on préconise une durée d'incubation comprise entre 2 et 4 heures, bornes incluses. Lorsque la durée d'incubation n'excède pas 2 heures, les composés Sug ne sont pas modifiés. Au delà de 2 heures, on observe des Sug réduit sous la forme de β-D-quinovosamine N-acétylée et/ou de β-D-fucosamine N-acétylée coexistant généralement avec des Sug non modifiés dans la chaîne polysaccharidique. Par conséquent, plus la durée d'incubation est importante, plus la proportion de Sug réduit est importante. Au delà de 4 heures, on identifie par RMN le composé X indésirable.

Afin de ne pas prolonger la durée de la réaction d'amination réductrice au-delà du temps requis, celle ci doit être arrêtée par des méthodes rapides, notamment par précipitation sélective du polysaccharide aminé. Le polysaccharide aminé est par exemple purifié par précipitation alcoolique lorsque le composé aminé est un agent de liaison ou un espaceur. On préfère la précipitation au sulfate d'ammonium, lorsque le composé aminé est un polypeptide. Les procédés de purification par précipitation sont des procédés classiques, bien connus de l'homme du métier.

L'amination réductrice est réalisée généralement dans un milieu tamponné tel que le tampon citrate / phosphate mais un autre tampon dont le pKa se situe entre 2,5 et 6 convient également. A titre d'exemple, on peut utiliser un tampon citrate, acétate ou succinate. On exclut cependant les tampons dont la formule chimique comporte des composés porteurs de groupement aminé, comme par exemple un tampon glycine:

La température du milieu réactionnel se situe en général entre 4 et 70°C selon le composé aminé utilisé, de façon préférée entre 20 et 50°C et de façon encore plus préférée entre 20 et 37°C lorsque le composé aminé est un polypeptide.

Pour usage dans le premier procédé selon l'invention, le polysaccharide peut être soit natif, soit fragmenté au préalable. N'importe quelle méthode de fragmentation peut convenir à condition qu'elle conserve l'intégrité structurale du polysaccharide natif. La méthode de fragmentation par radicaux libres, décrite plus loin, convient tout particulièrement.

Bien que les ratios pondéraux entre le polysaccharide, le composé aminé et le cyanoborohydrure ne soient pas critiques, on obtient de meilleurs rendements en polysaccharides aminés dans la gamme de ratios pondéraux polysaccharide / composé aminé / cyanoborohydrure allant de 1/0,1/0,02 à 1/5/0,2 lorsqu'on utilise comme composé aminé un agent de liaison L ou un espaceur S ; ou dans la gamme de ratios pondéraux allant de 1/ 0,2 / 0,02 à 1 / 1 / 0,2 lorsqu'on utilise comme composé aminé un polypeptide porteur P. Les rendements optima en polysaccharides aminés sont obtenus avec rapport 1/1/0,1 lorsque le composé aminé est un agent de liaison ou un espaceur ou avec un rapport 1/0,5/0,1 lorsque le composé aminé est un polypeptide porteur.

Pour usage dans le deuxième procédé selon l'invention, le polysaccharide est de préférence un polysaccharide natif, non dépolymérisé, puisque lors de la mise en oeuvre de ce deuxième procédé, le polysaccharide est nécessairement soumis à une fragmentation, après réduction, pour réintroduire des groupements aldéhyde terminaux.

Dans le deuxième procédé selon l'invention, la réduction du polysaccharide se fait de manière conventionnelle, *e.g*. à température ambiante, en milieu aqueux, à pH basique, de préférence à un pH compris entre 8 et 10. L'agent réducteur fort spécifique des fonctions cétone et aldéhyde est utilisé en excès *e.g*., à une concentration molaire d'au moins 10 fois, de façon préférée d'au moins 100 fois plus forte que celle du polysaccharide. Un borohydrure tel que le borohydrure de sodium (NaBH₄), est un agent réducteur de choix. Le temps réactionnel peut être de 30 min. à 2 heures, de préférence 1 heure. Le polysaccharide réduit peut être ensuite purifié par une méthode quelconque, de préférence par précipitation alcoolique.

La dépolymérisation du polysaccharide réduit peut se faire notamment par hydrolyse ménagée. On utilise de préférence la méthode de dépolymérisation radicalaire oxydative telle que décrite dans EP 562 107. Les polysaccharides fragmentés peuvent être ensuite purifiés de manière conventionnelle, *e.g*. par précipitation alcoolique avant l'étape d'amination réductrice.

Dans le deuxième procédé selon l'invention, l'amination réductrice peut être mise en oeuvre selon n'importe quelle condition, y compris les conditions classiques, car il n'existe plus de composé Sug dans la structure du polysaccharide réduit et fragmenté ; et par conséquent, plus de risque de former le composé X indésirable.

Ainsi, dans le deuxième procédé selon l'invention, l'amination réductrice à laquelle est soumis le polysaccharide requiert simplement la réaction du polysaccharide avec un composé comportant une fonction amine tel que les composés P, L ou S, en présence d'un agent réducteur, sélectif d'une base de Schiff.

On peut utiliser les conditions décrites pour le premier procédé selon l'invention ; et, comme dans ce cas la durée de l'incubation ainsi que le pH ne sont plus critiques, avec ou sans les limitations de durée ou les restrictions portant sur la gamme de pH. Généralement dans les conditions classiques, le pH du milieu réactionnel se situe dans une large gamme allant de 5 à 9, mais on choisit de préférence un pH compris entre 5 et 8. Un temps d'incubation généralement compris entre 2 heures et 48 heures donne de bons rendements en polysaccharide aminé. La température d'incubation est généralement comprise entre 20°C et 50°C.

Une fois l'amination réductrice menée à bien, on purifie le polysaccharide aminé du milieu réactionnel à l'aide de méthodes usuelles *e.g*. par précipitation alcoolique ou au sulfate d'ammonium, par dialyse, par chromatographie d'exclusion-diffusion ou par ultrafiltration, selon les besoins.

Ainsi que précédemment mentionné, un polysaccharide aminé peut être déjà un conjugué lorsque le composé ayant réagi avec la fonction aldéhyde terminale du polysaccharide est un polypeptide porteur. Ce conjugué est de formule Ps-*CH2-NH*-P, dans laquelle le polysaccharide (Ps) est directement couplé au polypeptide porteur (P).

C'est pourquoi l'invention a aussi pour objet :
a. Un procédé de fabrication d'un conjugué selon l'invention de formule (IV) Ps-*CH2-NH*-P dans laquelle Ps désigne le polysaccharide capsulaire du pneumocoque de type 5 ; procédé selon lequel on fait réagir le polysaccharide avec un polypeptide porteur (P), en présence d'un agent réducteur sélectif d'une base de Schiff, à un pH de 4 à 6,5, de préférence de 5 à 6, pendant une durée n'excédant pas 4 heures.
b. Un procédé de fabrication d'un conjugué selon l'invention de formule (IV) Ps-*CH2-NH*-P dans laquelle Ps désigne le polysaccharide capsulaire du pneumocoque de type 5 ; procédé selon lequel (i) on réduit le polysaccharide natif avec un agent réducteur fort spécifique des fonctions cétone et aldéhyde, (ii) on fragmente le polysaccharide réduit et (iii) on soumet le polysaccharide réduit et fragmenté à une amination réductrice en présence d'un polypeptide porteur P.
c. Un procédé de fabrication d'un conjugué selon l'invention de formule (V) Ps-*CH₂-NH-L*-P, selon lequel :
   (i) on fait réagir un polysaccharide capsulaire du pneumocoque de type 5 (Ps) avec un agent de liaison (L) possédant au moins une fonction amine primaire, en présence d'un agent réducteur sélectif d'une base de Schiff, à un pH de 4 à 6,5, de préférence de 5 à 6, pendant une durée n'excédant pas 4 heures, afin d'obtenir un polysaccharide activé de formule (VI) Ps-*CH2-NH*-L, et
   (ii) on couple le polysaccharide activé à un polypeptide porteur (P), afin d'obtenir le conjugué de formule (V) Ps-*CH2-NH-L*-P ; ou alternativement,
d. Un procédé de fabrication d'un conjugué selon l'invention de formule (V) Ps-*CH2-NH*-L-P, selon lequel on fait réagir un polysaccharide capsulaire du pneumocoque de type 5 (Ps) avec un polypeptide porteur activé de formule (VII) L-P, dans laquelle L est un agent de liaison possédant au moins une fonction amine primaire et P un polypeptide porteur, en présence d'un agent réducteur sélectif d'une base de Schiff, à un pH de 4 à 6,5, de préférence de 5 à 6, pendant une durée n'excédant pas 4 heures, afin d'obtenir le conjugué de formule (V) Ps-*CH2-NH*-L-P.
e. Un procédé de fabrication d'un conjugué selon l'invention, de formule (V) Ps-*CH2-NH*-L-P, selon lequel :
   (i) on fait réagir un polysaccharide capsulaire du pneumocoque de type 5 (Ps) avec un agent capable de réduire une fonction cétone,
   (ii) on fragmente le polysaccharide réduit,
   (ii) on couple par amination réductrice le polysaccharide réduit et fragmenté avec un agent de liaison (L) possédant au moins une fonction amine primaire afin d'obtenir un polysaccharide activé de formule (VI) Ps-*CH2-NH*-L, et
   on couple le polysaccharide activé à un polypeptide porteur (P), afin d'obtenir le conjugué de formule (V) Ps-*CH2-NH*-L-P; ou alternativement,
f. Un procédé de fabrication d'un conjugué selon l'invention, de formule (V) Ps-*CH2-NH*-L-P, selon lequel :
   (i) on fait réagir un polysaccharide capsulaire du pneumocoque de type 5 (Ps) avec un agent capable de réduire une fonction cétone,
   (ii) on fragmente le polysaccharide réduit, et
   (iii) on couple par amination réductrice le polysaccharide réduit et fragmenté avec un polypeptide porteur activé de formule (VII) L-P, dans laquelle L est un agent de liaison possédant au moins une fonction amine primaire, afin d'obtenir le conjugué de formule *(V)* Ps-*CH2-NH*-L-P.
g. Un procédé de fabrication d'un conjugué selon l'invention, de formule (VIII) Ps-*CH2-NH-*S-L'-P, selon lequel :
   (i) on fait réagir un polysaccharide capsulaire du pneumocoque de type 5 avec un espaceur (S) possédant au moins une fonction amine primaire, en présence d'un agent réducteur sélectif d'une base de Schiff, à un pH de 4 à 6,5, de préférence de 5 à 6, pendant une durée n'excédant pas 4 heures, afin d'obtenir un polysaccharide dérivatisé de formule (IX) Ps-*CH2-NH*-S,
   (ii) on couple le polysaccharide dérivatisé avec un agent de liaison (L') afin d'obtenir un polysaccharide activé de formule (X) Ps-*CH2-NH*-S-L', et
   (iii) on couple le polysaccharide activé avec un polypeptide porteur (P), afin d'obtenir le conjugué de formule (VIII) Ps-*CH2-NH*-S-L'-P ; ou alternativement,
h. Un procédé de fabrication d'un conjugué selon l'invention, de formule (VIII) Ps-*CH2-NH-*S-L'-P, selon lequel :
   (i) on fait réagir un polysaccharide capsulaire du pneumocoque de type 5 avec un espaceur (S) possédant au moins une fonction amine, en présence d'un agent réducteur sélectif d'une base de Schiff, à un pH de 4 à 6,5, de préférence de 5 à 6, pendant une durée n'excédant pas 4 heures, afin d'obtenir un polysaccharide dérivatisé de formule (IX) Ps-*CH2-NH*-S, et
   (ii) on couple le polysaccharide dérivatisé avec un polypeptide porteur activé de formule (XI) L'-P, dans laquelle L' est un agent de liaison et P un polypeptide porteur, afm d'obtenir le conjugué de formule (VIII) Ps-*CH2-NH*-S-L'-P*.*
i. Un procédé de fabrication d'un conjugué selon l'invention, de formule (VIII) Ps-*CH2-NH-*S-L'-P, selon lequel :
   (i) on fait réagir un polysaccharide capsulaire du pneumocoque de type 5 (Ps) avec un agent capable de réduire une fonction cétone,
   (ii) on fragmente le polysaccharide réduit,
   (iii) on couple par amination réductrice le polysaccharide réduit et fragmenté à un espaceur (S) porteur d'au moins une fonction amine primaire, afin d'obtenir un polysaccharide dérivatisé de formule (IX) Ps-*CH2-NH*-S,
   (iv) on couple le polysaccharide dérivatisé à un agent de liaison (L') afin d'obtenir un polysaccharide activé de formule (X) Ps-*CH2-NH*-S-L', et
   (v) on couple le polysaccharide activé à un polypeptide porteur (P), afin d'obtenir le conjugué de formule (VIII) Ps-*CH2-NH*-S-L'-P ; ou alternativement,
j. Un procédé de fabrication d'un conjugué selon l'invention, de formule (VIII) Ps-*CH2-NH* S-L'-P, selon lequel :
   (i) on fait réagir un polysaccharide capsulaire du pneumocoque de type 5 (Ps) avec un agent capable de réduire une fonction cétone,
   (ii) on fragmente le polysaccharide réduit,
   (iii) on couple par amination réductrice le polysaccharide réduit et fragmenté à un espaceur (S) porteur d'au moins une fonction amine primaire, afin d'obtenir un polysaccharide dérivatisé de formule (IX) Ps-*CH2-NH*-S, et
   (iv) on couple le polysaccharide dérivatisé avec un polypeptide porteur activé de formule (XI) L'-P, dans laquelle L' est un agent de liaison et P un polypeptide porteur, afm d'obtenir le conjugué de formule (VIII) Ps-*CH2-NH*-S-L'-P.

Dans sa portée la plus générale, le terme de polypeptide porteur (P) désigne une chaîne d'acides aminés, quelque soit sa taille et les modifications post-traductionelles qui ont pu se produire, comprenant au moins un épitope « T-helper ». Sachant qu'un épitope T-helper peut être constitué par 10-15 acides aminés, le terme de polypeptide porteur englobe les peptides. Bien évidemment, il englobe aussi les protéines.

Par épitope « T-helper », on entend un enchaînement d'acides aminés qui, dans le contexte d'une ou plusieurs molécules du MHC, active les lymphocytes T-helper. Le polypeptide porteur du conjugué provoque le développement d'une immunité T-dépendante spécifique du polysaccharide du pneumocoque de type 5 avec production d'anticorps spécifiques dirigés contre le polysaccharide à la suite de l'administration du conjugué. Il induit également une élévation du taux d'anticorps spécifiques lors d'une vaccination de rappel.

Pour usage dans les conjugués selon l'invention, le polypeptide porteur peut être une anatoxine bactérienne obtenue par détoxification chimique, comme l'anatoxine tétanique ou obtenue par mutation génétique comme l'anatoxine diphtérique (CRM 197, à titre d'exemple), l'exoprotéine A de *Pseudomonas aeruginosa* ou l'exotoxine A de *Staphylococcus aureus,.* On peut également utiliser les protéines de membrane externe des bactéries, comme les protéines OMP1 ou OMP2 de *Neisseria meningitidis,.* Les protéines lambB, OmpC, OmpA, OmpF et PhoE d'*Escherichia coli,* la protéine CotC ou CotD de *Bacillus subtilis,* les porines bactériennes comme la porine de classe 1 de *Neisseria meningitidis B,* la porine P40 de *Klebsiella pneumoniae ;* également les lipoprotéines comme OspA de *Borrelia burgdorfi,* PspA de *Streptococcus pneumoniae,* la Transferrine binding protein type 2 (TBP2) de *Neisseria meningitidis,* TraT d'*Escherichia coli,* l'adhésine A de *Streptococus pneumoniae.* Les protéines d'origine virale, comme l'hémagglutinine du virus grippal peuvent également jouer le rôle de polypeptide porteur ainsi que les peptides p24E, p 24N, p 24M, p24H décrits dans WO 94/29339 portant un épitope T helper, ou les peptides PADRE (PanDR T helper epitopes) décrits par Del guercio et al (Vaccine (1997), vol 15/4, p441-448).

La fabrication des conjugués de formule (IV) Ps-*CH2-NH*-P se réalise en une seule étape, la réaction d'amination réductrice du polysaccharide assurant également la conjugaison du polysaccharide au polypeptide. Par contre, la fabrication des conjugués de formule (V) Ps-*CH2-NH-*L-P ou (VIII) Ps-*CH2-NH*-S-L'-P se réalise en plusieurs étapes distinctes, la première étape étant constituée par la mise en oeuvre de l'un des deux procédés de fabrication d'un polysaccharide aminé selon l'invention aboutissant à l'amination réductrice du polysaccharide par le composé L ou S.

L'espaceur S et les agents de liaison L et L'sont des composés possédant au moins deux groupements fonctionnels et disposés au sein du composé, selon des directions relativement opposées. En ce qui concerne l'espaceur S et l'agent de liaison L, l'un des groupements fonctionnels doit être capable de réagir avec l'aldéhyde terminal du polysaccharide lors de l'amination réductrice, l'autre étant respectivement capable de réagir avec un agent de liaison L' ou avec un polypeptide porteur. En ce qui concerne l'agent de liaison L', l'un des groupements fonctionnels doit être capable de réagir avec l'espaceur, l'autre étant capable de réagir avec un polypeptide porteur.

Aux fins de la présente invention, l'agent de liaison L est un composé de formule (XII) R1-A-R2, dans laquelle :
A désigne une chaîne aliphatique, aromatique, ou une chaîne mixte aliphatique et aromatique, substituée ou non, saturée ou insaturée ;
R1 désigne une amine primaire ou un radical chimique porteur d'une amine primaire, comme par exemple le radical hydrazide, NH2-NH-CO ; et
R2 désigne un groupement fonctionnel capable de réagir avec un groupement fonctionnel du polypeptide porteur.

De manière avantageuse, A désigne un alkyl, un alkylène ou un dithioalkyl, comprenant de 1 à 12, avantageusement de 2 à 8, de préférence de 2 à 6 atomes de carbone ;

De manière avantageuse, R2 est capable de réagir avec un groupement carboxyl, thiol ou amine. Ainsi R2 peut être indépendamment de R1, un groupement amine ou un radical porteur d'un groupement amine tel que le radical hydrazide. R2 peut aussi être un groupement thiol ou carboxyl.

Ainsi, un composé de formule R1-A-R2 peut être un alkyl dihydrazide tel que l'acide adipique dihydrazide ; un thio alkyl monoaminé tel que la cystéine ou la cystéamine, ou diaminé tel que la cystamine ; un diamino alkyl ou alkylène, tel que le diaminométhane, le diamino ethane et le diaminohexane.

Aux fins de la présente invention, l'espaceur S est un composé de formule (XIII) R1-A-R2', dans laquelle :
A désigne une chaîne aliphatique et/ou aromatique, substituée ou non, saturée ou insaturée ;
R1 désigne une amine primaire ou un radical chimique porteur d'une amine primaire, comme par exemple le radical hydrazide, NH2-NH-CO ; et
R2' désigne un groupement fonctionnel capable de réagir avec un groupement fonctionnel d'un agent de liaison L'.

Selon un mode particulier, l'espaceur S peut être choisi parmi les composés de formule (XII) R1-A-R2.

Aux fins de la présente invention, le choix de l'agent de liaison L' est conditionné en premier lieu par le groupement fonctionnel R2' porté par S puis par le groupement fonctionnel du polypeptide porteur devant intervenir dans l'opération de conjugaison. L'agent de liaison L' est un composé de formule (XIV) R3-B-R4, dans laquelle :
B désigne une chaîne aliphatique et/ou aromatique, substituée ou non, saturée ou insaturée ;
R3 désigne un groupement fonctionnel capable de réagir avec le groupement fonctionnel R2' ; et
R4 désigne un groupement fonctionnel capable de réagir avec un groupement fonctionnel du polypeptide porteur.

De manière avantageuse, B désigne un alkyl ou un alkylène substitué ou non, comprenant de 1 à 12, de façon préférée de 2 à 8 atomes de carbone ; un aryl, un alkylaryl ou un arylalkylène comprenant de 7 à 12 atomes de carbone ; un phényl ou phénylène substitué ou non.

Lorsque R2'est un groupement thiol, R3 peut être un groupement thiol ; un carbonyl α ou β insaturé ou un groupe imidyl, en particulier un groupe maleimidyl ; un halogénure d'acyle ou un halogénure d'alkyle, dans lequel l'halogène est un brome, un chlore, ou un iode.

De manière avantageuse, R4 est capable de réagir avec un groupement carboxyl, thiol ou amine. Ainsi, si le groupement fonctionnel du polypeptide porteur devant intervenir dans l'opération de conjugaison est un thiol, R4 peut être un groupement maléimide. De même, si le groupement fonctionnel du polypeptide porteur devant intervenir dans l'opération de conjugaison est une amine, R4 peut être un groupement carboxyl ou de préférence un groupement N-hydroxy succinimidyl ou N-hydroxy sulfosuccinimidyl.

Lorsque l'espaceur S est un aminothiol tel que la cystéine, la cystéamine, ou la cystamine, l'agent de liaison L'est de manière avantageuse un sucinimidyl maleimidyl alkyl. Ce dernier peut être notamment l'acide y maleimido butyrique N-hydroxyssuccinimide ester ou N-sulfosuccinimide ester (GMBS ou sulfo-GMBS), l'acide ε maleimido caproique N-hydroxyssuccinimide ester (MCS), le succinimidyl-4-(p-maleimidophenyl)-butyrate (SMPB) ou le sulfosuccinimidyl-4-(p-maleimidophenyl)-butyrate (sulfo-SMPB), l'acide maleimido benzoïque N-hydroxyssuccinimide ester (MBS) ou l'acide maleimido benzoïque N-hydroxysulfosuccinimide ester (sulfo-MBS), l'acide 4-(N-maleimidomethyl) cyclohexane carboxylique N-hydroxyssuccinimide ester (SMCC) ou l'acide 4-(N-maleimidomethyl) cyclohexane carboxylique N-hydroxysulfosuccinimide ester (sulfo-SMCC).

Lorsque l'espaceur S est un diaminoalkyl ou un dihydrazide, l'agent de liaison L' est avantageusement choisi parmi les composés dissucinimidyl alkyls ou succinimidyl maleimido alkyls de formule (XIV) R3-B-R4 dans laquelle B est un groupement alkyl, R3 est un groupement succinimidyl et R4 est un groupement succinimidyl ou maleimido.

Le composé disuccinimidyl peut être le disuccinimidyl subérate (DSS), le bis (sulfosuccinimidyl) suberate (BS3), le dissucinimidyl glutarate (DSG), le succinimidyldiester de l'acide adipique (SIDEA) ou le succinimidyldiester de l'acide succinique. Les groupements succinimidyls et ou sulfo succinimidyl sont capables de réagir avec un groupement amine. Le composé succinimidyl maleimido alkyl peut être un de ceux cités plus haut.

Dans les procédés de conjugaison a. à j., les étapes de dérivatisation, d'activation et de conjugaison proprement dites peuvent être mises en oeuvre selon des modes opérationnels bien connus de l'homme du métier. Ils sont notamment décrits dans l'ouvrage de référence intitulé Bioconjugate Techniques (1996) Ed Academic press. A titre d'exemple, on se réfère à cet ouvrage pour indiquer que des réactions de conjugaison mettant en jeu un groupement amine et un groupement carboxyl se réalisent avantageusement en présence d'une carbodiimide.

Les étapes de dérivatisation, d'activation et de conjugaison sont sans effet sur la structure chimique des unités répétitives du polysaccharide.

Les conjugués obtenus selon l'un des procédés a. à j. peuvent être finalement purifiés *e.g*. par précipitation au sulfate d'ammonium, par ultrafiltration, chromatographie d'exclusion-diffusion ou par chromatographie de partage de façon à éliminer les fractions polysaccharidique et protéique résiduelles non conjuguées.

L'invention a également pour objet une composition pharmaceutique pour un usage thérapeutique ou prophylactique, comprenant un polysaccharide selon l'invention, de façon préférée sous la forme d'un conjugué. Ce dernier peut être formulé avec un diluant ou un support acceptable d'un point de vue pharmaceutique *e.g*. un tampon phosphate, et le cas échéant un excipient de lyophilisation. En général, ces produits peuvent être sélectionnés en fonction du mode et de la voie d'administration et selon les pratiques pharmaceutiques standard. Les diluants appropriés ainsi que ce qui est indispensable à l'élaboration d'une composition pharmaceutique sont décrits dans *Remington's Pharmaceutical Sciences,* servant de référence standard dans ce domaine. La composition peut aussi contenir un adjuvant *e.g*., un hydroxyde d'aluminium, un phosphate d'aluminium ou un hydroxyphosphate d'aluminium. On peut également utiliser un conservateur tel que le phenoxyéthanolformol. Une dose vaccinale peut être préparée sous un volume de 0,1 ml à 2 ml, de façon préférée sous un volume de 0,5 ml. A titre d'exemple on indique qu'une dose peut contenir 0,475 mg d'ion PO₄²⁻, 4,5 mg de chlorure de sodium et éventuellement 300 µg d'ions AL³⁺. La composition vaccinale selon l'invention peut être également associée à d'autres antigènes vaccinaux, notamment des antigènes polysaccharidiques du pneumocoque conjugués ou non à un porteur polypeptidique. On obtient alors un vaccin multivalent contre le pneumocoque dans lequel le polysaccharide du pneumocoque type 5 est sous l'une des formes décrites dans l'invention.

L'invention a aussi pour objet une méthode de traitement ou de prévention contre les infections à pneumocoque type 5, qui consiste à administrer à des nourrissons, des jeunes enfants, des adultes ou des personnes âgées, une dose suffisante d'une composition selon l'invention éventuellement adjuvantée, pour induire une réponse immune spécifique protectrice contre ce pathogène. La méthode est mise en oeuvre par administration d'au moins une dose vaccinale de la composition selon l'invention. Par exemple, on peut pratiquer entre 1 et 3 injections, mais de préférence on pratique 3 injections en respectant un délai d'un mois entre chaque injection. Une composition selon l'invention peut être administrée par n'importe quelle voie conventionnelle en usage dans le domaine des vaccins, en particulier par voie systémique *i.e*., parentérale *e.g*., par voie sous cutanée, intramusculaire, intradermique ou intraveineuse; ou par voie muqueuse *e.g*., par voie orale ou nasale. La quantité administrée tient compte du polypeptide porteur utilisé ou de la voie d'administration. A titre d'exemple, la dose de polysaccharide nécessaire contenue dans le conjugué pour observer une immunité protectrice vis à vis du sérotype 5 consécutive à une administration parentérale est comprise généralement entre 0,5 µg et 10 µg; mais de façon préférentielle entre 0,5 et 5 µg, et de façon encore plus préférée entre 0,5 µg et 2 µg lorsque la protéine porteuse est l'anatoxine tétanique. Sous une forme non conjuguée, la dose de polysaccharide nécessaire est comprise entre 10 et 50 µg, de façon préférée entre 20 et 30 µg.

La présente invention sera mieux comprise à la lumière des exemples suivants qui servent à illustrer l'invention sans pour autant en limiter le contenu.

La figure 1 représente les spectres RMN d'un polysaccharide aminé selon différents procédés de fabrication.

Le premier spectre est celui du polysaccharide du pneumocoque type 5 fragmenté après dépolymérisation oxydative radicalaire. On identifie dans la zone des hauts champs du spectre les signaux de résonance des carbones méthyliques des 3 hexosamines N-acétylés de l'unité répétitive du polysaccharide : la fucosamine N acétylée (C6 FucNAc), la pneumosamine N acétylé (C6 PneNAc) et le Sug (C6 Sug).

Le deuxième spectre est celui du polysaccharide aminé obtenu selon le second procédé objet de l'invention. On observe la disparition du signal correspondant au Sug (C6 Sug) et l'apparition d'un nouveau signal correspondant à la quinovosamine N acétylée (C6 QuiNAc).

Le troisième spectre est celui du polysaccharide aminé obtenu selon le premier procédé objet de l'invention. Il est identique au spectre RMN du polysaccharide fragmenté non aminé.

Le quatrième spectre est celui du polysaccharide aminé dans des conditions classiques. On note la présence de deux signaux additionnels dans la zone des hauts champs du spectre, correspondant à la quinovosamine N acétylé et au composé X ainsi qu'à une diminution de la hauteur du signal correspondant au Sug.

La figure 2 représente le chromatogramme obtenu par HPAEC-PAD du polysaccharide natif dépolymérisé (courbe à trait continu), du polysaccharide aminé selon le procédé classique d'amination réductrice (courbe en tiret cadratin), selon le premier procédé objet de l'invention (courbe en tiret) ou selon le second procédé objet de l'invention (courbe en tiret point).

### Exemple 1 : Amination réductrice à pH 6, pendant 2 heures, du polysaccharide capsulaire du pneumocoque type 5, après fragmentation.

### a) Dépolymérisation radicalaire du polysaccharide du pneumocoque type 5 natif

Le polysaccharide à une concentration de 2,5 mg/ml en solution aqueuse est fragmenté par de l'acide ascorbique, du sulfate ferreux ainsi que du sulfate cuivrique. La quantité en nombre de mmoles d'acide ascorbique est cent fois supérieure à celle du sulfate ferreux et du sulfate cuivrique. Le rapport pondéral entre l'acide ascorbique et le polysaccharide est de 0,1. L'incubation du mélange réactionnel se fait en bain-marie à 30°C et à l'abri de la lumière pendant 1 heure 30. Le polysaccharide hydrolysé est purifié par précipitation dans l'éthanol à 80 % suivie d'une centrifugation. Le culot de centrifugation est dialysé puis lyophilisé. La taille moyenne du polysaccharide fragmenté est d'environ 30-35 unités répétitives, telle que mesurée par chromatographie d'exclusion à triple détection (Viscoteck).

### b) Amination réductrice du polysaccharide

Le polysaccharide fragmenté est remis en solution dans un tampon citrate/phosphate 0,2 M, pH 6 à une concentration de 10 mg/ml en présence de chlorhydrate de diaminohexane (DAH) (Aldrich) et de cyanoborohydrure de sodium (NaCNBH₃) (Sigma). Les rapports pondéraux polysaccharide/chlorhydrate de DAH et NaCNBH₃/chlorhydrate de DAH sont respectivement de 0,8 et 0,1. L'incubation du mélange réactionnel se fait à 50°C en bain-marie pendant 2 heures. La réaction est ensuite stoppée par précipitation du polysaccharide aminé dans de l'éthanol à 80 % suivie d'une centrifugation. Le culot de précipitation est ensuite repris par du NaCl 0,5 M à raison de 10 mg/ml en polysaccharide puis soumis à 8 bains de dialyse successifs (4 premiers bains effectués dans une solution de NaCl 0,5 M suivis de 4 bains effectués dans de l'eau ultra-filtrée). Le polysaccharide dérivatisé et ainsi aminé est finalement lyophilisé.

### Exemple 2 : Amination réductrice du polysaccharide capsulaire du pneumocoque de type 5, après réduction et fragmentation.

### a) Réduction et fragmentation du polysaccharide natif

A 10 ml d'une solution aqueuse de polysaccharide natif à 10 mg/ml ajustée à pH 9 ± 0,5 avec de l'ammoniaque 1 N, on ajoute 10 mg de borohydrure de sodium (NaBH₄), puis on laisse le milieu réactionnel à température ambiante pendant 2 heures à l'obscurité. Le NaBH4 est ensuite détruit par addition de quelques gouttes d'acide acétique glacial. On dialyse ensuite le polysaccharide réduit contre de l'eau puis on le fragmente en utilisant le protocole décrit dans le paragraphe a) de l'exemple 1. On obtient un polysaccharide contenant en moyenne 30-35 unités répétitives. Le polysaccharide réduit et fragmenté est par la suite lyophilisé.

### b) Amination réductrice du polysaccharide réduit et fragmenté

Le polysaccharide réduit et fragmenté est remis en solution dans un tampon phosphate 0,2 M, pH 7,5 à une concentration de 10 mg/ml en présence de chlorhydrate de diaminohexane (DAH) et de cyanoborohydrure de sodium (NaCNBH₃) préparé extemporanément. Les rapports pondéraux polysaccharide/chlorhydrate de DAH et NaCNBH₃/chlorhydrate de DAH sont respectivement de 0,8 et 0,1. L'incubation du mélange réactionnel se fait à 50°C en bain-marie pendant 48 à 72 heures. Le polysaccharide aminé est ensuite purifié par précipitation dans de l'éthanol à 80 % suivie d'une centrifugation. Le culot de précipitation est repris par du NaCl 0,5 M à raison de 10 mg/ml en polysaccharide puis soumis à 8 bains de dialyse successifs (4 premiers bains effectués dans une solution de NaCl 0,5 M suivis de 4 bains effectués dans de l'eau ultra-filtrée). Le polysaccharide dérivatisé et ainsi aminé est finalement lyophilisé.

### Exemple 3 : Fabrication et étude du pouvoir immunogène des conjugués obtenus à partir des polysaccharides dérivatisés et aminés des exemples 1 et 2.

### a) Fabrication des conjugués.

Les polysaccharides aminés des exemples 1 et 2 sont conjugués à l'anatoxine tétanique (ATT) par l'intermédiaire du DSS. Dans un premier temps, on active le polysaccharide aminé avec du DSS puis on conjugue l'anatoxine tétanique au polysaccharide activé.

Le polysaccharide aminé de l'exemple 1 ou 2 est repris en solution aqueuse à une concentration de 40 mg/ml. Le DSS est repris dans une solution de DMSO à raison de 5 mg/ml. On ajoute goutte à goutte 2,5 ml d'une solution aqueuse du polysaccharide aminé dans 10 ml de la solution de DSS sous agitation. Après 1 h 30 d'incubation, la réaction est stoppée par précipitation dans 50 ml d'acétone. On récupère le précipité par filtration sur büchner n°5, que l'on lave ensuite 5 fois par 20 à 50 ml d'acétone. On sèche ensuite le précipité sous vide puis sous courant d'azote.

Le précipité contenant le polysaccharide dérivatisé est repris par un volume adéquat d'une solution d'anatoxine tétanique à 10 mg/ml dans un tampon phosphate 0,2 M de sorte que le ratio pondéral entre le polysaccharide dérivatisé et l'anatoxine tétanique soit compris entre 1 et 2. Le mélange est incubé à la température du laboratoire, sous agitation pendant 16 à 20 heures. La réaction est ensuite stoppée par précipitation au sulfate d'ammonium à 70 % après dilution au ¼ du mélange dans un tampon phosphate 0,2 M. Le milieu contenant le précipité est agité pendant 1 heure à la température du laboratoire puis pendant 4 à 20 heures à +4°C. Après avoir recueilli le précipité par centrifugation, celui ci est finalement repris et dissous dans un tampon phosphate 0,2 M. La solution du conjugué est obtenue sous une forme pratiquement pure. Les quantités d'anatoxine tétanique et de polysaccharide non conjugués sont négligeables. La solution est finalement ajustée à la concentration désirée en vue d'une utilisation vaccinale. Le ratio polysaccharide /protéine dans le conjugué est de 1/5.

### b) Etude du pouvoir immunogène des conjugués

Le pouvoir immunogène des conjugués ainsi obtenus est comparé avec celui d'un conjugué faisant usage d'un polysaccharide capsulaire du pneumocoque de type 5, préalablement soumis à un amination réductrice classique, décrite comme suit : Le polysaccharide natif est tout d'abord fragmenté par dépolymérisation radicalaire selon le protocole opératoire du paragraphe a) de l'exemple 1. On obtient un polysaccharide dépolymérisé contenant 30-35 unités répétitives. Puis le polysaccharide fragmenté est soumis à une amination réductrice selon le protocole opératoire du paragraphe b) de l'exemple 2. Le polysaccharide aminé est ensuite conjugué à l'anatoxine tétanique par l'intermédiaire du DSS dans les conditions opératoires du paragraphe a) de l'exemple 3. Le conjugué ainsi obtenu a les mêmes caractéristiques de pureté et est utilisable comme vaccin. Le ratio polysaccharide /protéine dans le conjugué est de 1/5.

Quinze lapins NZB répartis dans 3 groupes distincts reçoivent par voie intramusculaire aux jours J1 et J23 l'un des 3 conjugués polysaccharidiques à raison de 0,5 µg de polysaccharide et de 2,5 µg d'anatoxine tétanique par lapin et par injection. Le groupe 1 est immunisé avec le conjugué obtenu à partir du polysaccharide aminé de l'exemple 1, le groupe 2 avec le conjugué obtenu à partir du polysaccharide aminé de l'exemple 2, le groupe 3 avec le conjugué obtenu à partir du polysaccharide aminé selon un procédé classique tel que décrit dans le paragraphe précédent du présent exemple. Un groupe de lapins « témoin » reçoit à J1 et J23 du sérum physiologique par voie intramusculaire.

Des prélèvements de sang sont réalisés à J1, J23 et J36 pour contrôler les taux d'anticorps sériques qui sont spécifiques du polysaccharide du pneumocoque type 5 par ELISA sur des microplaques sensibilisées avec du polysaccharide natif de pneumocoque type 5 (1 µg/micropuits). Les taux d'anticorps contenus dans chaque lapin sont définis comme étant l'inverse de la dilution du sérum qui donne une densité optique de 1 au spectrophotomètre à la suite de la révélation du test ELISA à l'aide d'un indicateur coloré le trimethyl benzidine. Les résultats sont regroupés dans le tableau I ci-après :

| Groupe | J1 | J23 | J36 |
|---|---|---|---|
| 1 | **<10** | **239***(122-469)** | **2824**(1758-4535) |
| 2 | **<10** | **110** (74-164) | **2929** (1180-7270) |
| 3 | **<10** | **57**(29-113) | **423**(166-1078) |
| Témoin | **<10** | **<20** | **<20** |

| | | | |
|---|---|---|---|
| *: représente la moyenne géométrique des taux d'anticorps sériques spécifiques des 5 lapins **: indique la valeur du taux d'anticorps le plus bas et la valeur du taux le plus élevée dans chaque groupe. | | | |

Après deux immunisations, les valeurs moyennes des taux d'anticorps spécifiques des lapins immunisés avec les conjugués obtenus à partir des polysaccharides aminés des exemples 1 et 2 sont environ 5 fois supérieures à celle des lapins immunisés avec un conjugué obtenu à partir d'un polysaccharide aminé selon un procédé classique d'amination réductrice.

Lorsque l'on fait varier les ratios pondéraux polysaccharide/ anatoxine tétanique dans une gamme allant de 1/0,5 à 1/5, les résultats que l'on obtient dans le test précédemment décrit sont en tout point similaire.

La fonctionnalité de ces anticorps est contrôlée par un test d'opsonophagocytose. Le titre opsonisant est défini comme étant l'inverse de la dilution de sérum qui permet de tuer 50 % des bactéries. Les résultats sont regroupés dans le tableau II ci-après :

| Groupe | J1 | J23 | J36 |
|---|---|---|---|
| 1 | **n.t.** | **7*** (3-18)** | **128** (27-599) |
| 2 | **n.t.** | **2** (2-3) | **185** (60-565) |
| 3 | **n.t.** | **2** (1-5) | **35** (11-110) |
| Témoin | **n.t.** | **n.t.** | **n.t.** |

| | | | |
|---|---|---|---|
| *: représente la moyenne géométrique des titres opsonisants des 5 lapins **: indique la valeur du titre opsonisant le plus bas et la valeur du titre le plus élevée dans chaque groupe. n.t. : non titré | | | |

Les titres opsonisants corrèlent avec les titres anticorps sériques spécifiques du polysaccharide du pneumocoque type 5.

## Revendications

1. Un polysaccharide capsulaire du pneumocoque de type 5 aminé au niveau du groupement aldéhyde terminal, constitué d'unités répétitives, dont au moins 85 % des unités répétitives répondent à la formule (II) 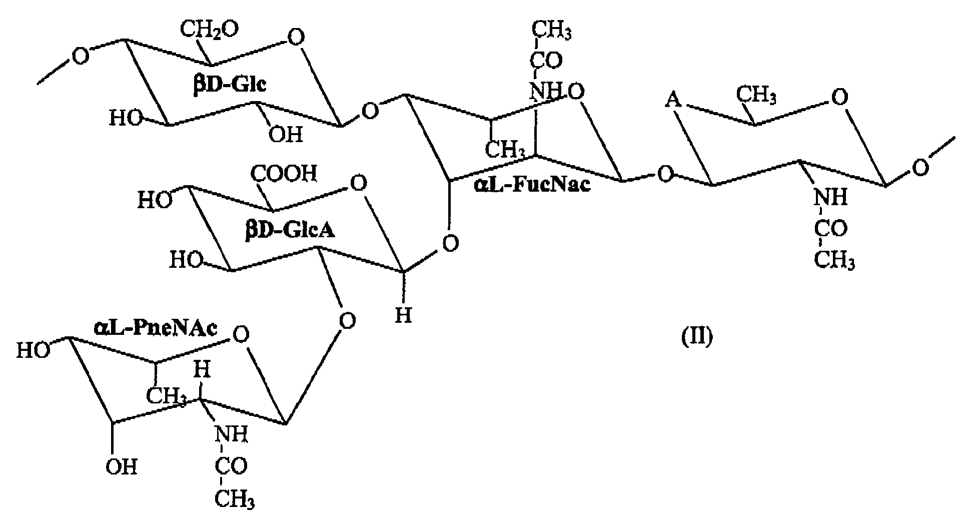 dans laquelle A est de manière indépendante et aléatoire C=O ou CHOH.

2. Un polysaccharide selon la revendication 1, dans lequel au moins 90 % des unités répétitives répondent à la formule (II).

3. Un polysaccharide selon la revendication 2, dans lequel au moins 95 % des unités répétitives répondent à la formule (II).

4. Un polysaccharide selon l'une des revendications 1 à 3, dans lequel au moins 95 % des unités répétitives répondant à la formule (II) répondent à la formule II" 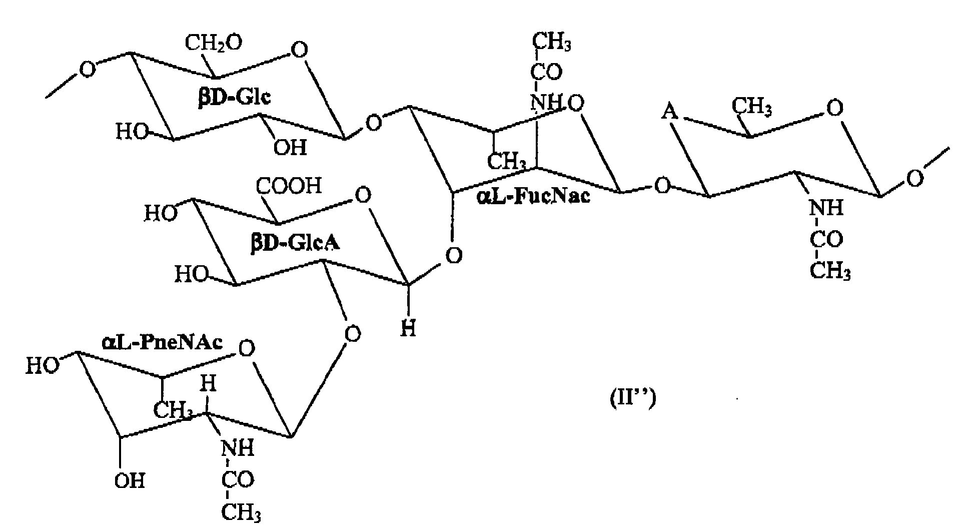 dans laquelle A est CHOH.

5. Un polysaccharide selon l'une des revendications 1 à 3, dans lequel 85 à 95 % des unités répétitives répondant à la formule (II) répondent à la formule II' 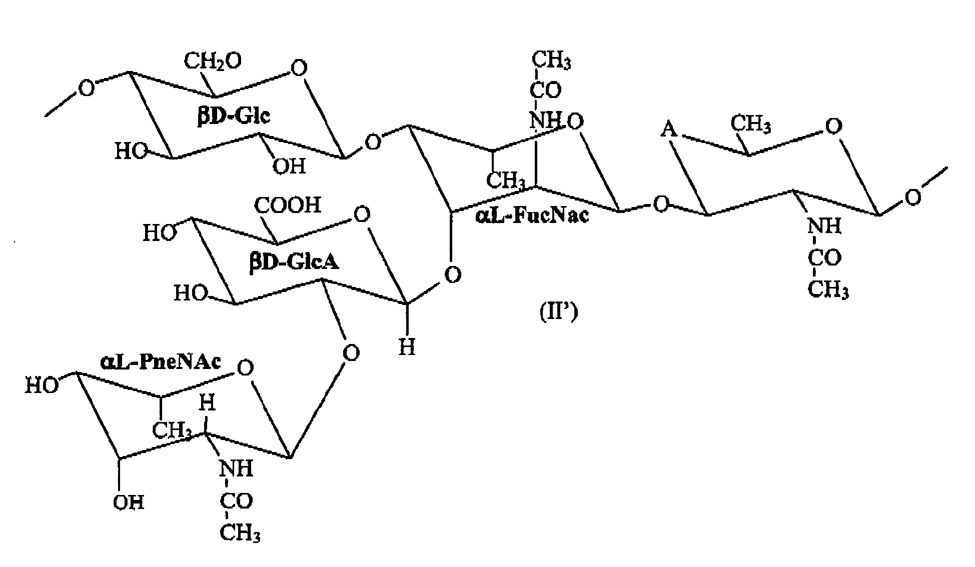 dans laquelle A est C=O.

6. Un conjugué dans lequel un polysaccharide selon l'une des revendications 1 à 5 est couplé à un polypeptide porteur (P).

7. Un procédé de fabrication d'un polysaccharide capsulaire du pneumocoque de type 5 aminé, selon lequel on soumet le polysaccharide à une amination réductrice en présence d'un agent réducteur sélectif d'une base de Schiff, à un pH de 4 à 6.5, pendant une durée n'excédant pas 4 heures.

8. Un procédé selon la revendication 7, dans lequel on soumet le polysaccharide à une amination réductrice à un pH de 5 à 6.

9. Un procédé selon la revendication 7 ou 8, dans lequel on soumet le polysaccharide à une amination réductrice pendant une durée n'excédant pas 2 heures.

10. Un procédé selon la revendication 7, 8 ou 9, dans lequel l'agent réducteur sélectif d'une base de Schiff est le cyanoborohydrure ou le complexe pyridine borane.

11. Un procédé de fabrication d'un polysaccharide capsulaire du pneumocoque de type 5 aminé, selon lequel (i) on fait réagir le polysaccharide avec un agent capable de réduire une fonction cétone, (ii) on fragmente le polysaccharide réduit et (iii) on soumet le polysaccharide réduit et fragmenté à une amination réductrice.

12. Un procédé selon la revendication 11, dans lequel le polysaccharide que l'on fait réagir avec l'agent capable de réduire une fonction cétone est sous forme native.

13. Un procédé selon la revendication 11 ou 12, dans lequel l'agent capable de réduire une fonction cétone est le NaBH₄.

14. Un procédé selon la revendication 11, 12 ou 13, dans lequel on fragmente le polysaccharide réduit par dépolymérisation radicalaire oxydative.

15. Un procédé de fabrication d'un conjugué de formule Ps-*CH2-NH*-P, selon lequel on met en oeuvre un procédé selon l'une des revendications 7 à 10, dans lequel le polysaccharide capsulaire du pneumocoque de type 5 (Ps) est couplé par amination réductrice avec un polypeptide porteur (P), en présence d'un agent réducteur sélectif d'une base de Schiff, à un pH de 4 à 6,5, de préférence de 5 à 6, pendant une durée n'excédant pas 4 heures.

16. Un procédé de fabrication d'un conjugué de formule Ps-*CH2-NH*-P, selon lequel on met en oeuvre un procédé selon l'une des revendications 11 à 14, dans lequel le polysaccharide capsulaire du pneumocoque de type 5 (Ps) réduit et fragmenté est couplé par amination réductrice avec un polypeptide porteur (P) :

17. Un procédé de fabrication d'un conjugué de formule Ps-*CH2-NH*-L-P, selon lequel :
(i) on met en oeuvre le procédé selon l'une des revendications 7 à 10, dans lequel le polysaccharide capsulaire du pneumocoque de type 5 (Ps) est couplé par amination réductrice à un agent de liaison (L) possédant au moins une fonction amine primaire, pour former un polysaccharide aminé et activé de formule Ps-*CH2-NH*-L, et on couple le polysaccharide activé à un polypeptide porteur (P) afin d'obtenir le conjugué de formule Ps-*CH2-NH*-L-P ; ou alternativement,
(ii) on met en oeuvre le procédé selon l'une des revendications 7 à 10, dans lequel le polysaccharide capsulaire du pneumocoque de type 5 (Ps) est couplé par amination réductrice à un polypeptide porteur activé de formule L-P, dans laquelle L est un agent de liaison possédant au moins une fonction amine primaire, afin d'obtenir le conjugué de formule Ps-*CH2-NH*-L-P.

18. Un procédé de fabrication d'un conjugué de formule Ps-*CH2-NH*-L-P, selon lequel :
(i)
a) on met en oeuvre le procédé selon l'une des revendications 11 à 14, dans lequel le polysaccharide capsulaire du pneumocoque de type 5 (Ps) réduit et fragmenté est couplé par amination réductrice à un agent de liaison (L) possédant au moins une fonction amine primaire, pour former un polysaccharide aminé et activé de formule Ps-*CH2-NH*-L, et
b) on couple le polysaccharide activé à un polypeptide porteur (P) afin d'obtenir le conjugué de formule Ps-*CH2-NH*-L-P ; ou alternativement,
(ii) on met en oeuvre le procédé selon l'une des revendications 11 à 14, dans lequel le polysaccharide capsulaire du pneumocoque de type 5 (Ps) réduit et fragmenté est couplé par amination réductrice à un polypeptide porteur activé de formule L-P, dans laquelle L est un agent de liaison possédant au moins une fonction amine primaire, afin d'obtenir le conjugué de formule Ps-*CH2-NH*-L-P.

19. Un procédé de fabrication d'un conjugué de formule Ps-*CH2-NH*-S-L'-P, selon lequel :
(i) a) on met en oeuvre le procédé selon l'une des revendications 7 à 10, dans lequel le polysaccharide capsulaire du pneumocoque de type 5 (Ps) est couplé par amination réductrice à un espaceur (S) possédant au moins une fonction amine primaire, pour former un polysaccharide aminé et dérivatisé de formule Ps-*CH2-NH*-S, et b) on couple le polysaccharide dérivatisé avec un agent de liaison (L') afin d'obtenir un polysaccharide activé de formule Ps-*CH2-NH*-S-L', puis on couple le polysaccharide activé avec polypeptide porteur (P) afin d'obtenir le conjugué de formule Ps-*CH2-NH*-S-L'-P ; ou alternativement,
(ii) b) on couple le polysaccharide dérivatisé, avec un polypeptide porteur activé de formule L'-P, dans laquelle L' est un agent de liaison, afin d'obtenir le conjugué de formule Ps-*CH2-NH-*S-L'-P.

20. Un procédé de fabrication d'un conjugué de formule Ps-*CH2-NH*-S-L'-P, selon lequel :
(i)
a) on met en oeuvre le procédé selon l'une des revendications 11 à 14, dans lequel le polysaccharide capsulaire du pneumocoque de type 5 (Ps) réduit et fragmenté est couplé par amination réductrice
à un espaceur (S) possédant au moins une fonction amine primaire, pour former un polysaccharide aminé et dérivatisé de formule Ps-*CH2-NH*-S, et
b) on couple le polysaccharide dérivatisé avec un agent de liaison (L') afin d'obtenir un polysaccharide activé de formule Ps-*CH2-NH*-S-L', puis on couple le polysaccharide activé avec un polypeptide porteur (P), afin d'obtenir le conjugué de formule Ps-*CH2-NH*-S-L'-P ; ou alternativement,
(ii) b) on couple le polysaccharide dérivatisé, avec un polypeptide porteur activé de formule L'-P, dans laquelle L' est un agent de liaison, afin d'obtenir le conjugué de formule Ps-*CH2-NH*-S-L'-P.

21. Un procédé selon l'une des revendications 15 à 20, dans lequel le polypeptide porteur P est l'anatoxine diphtérique ou tétanique.

22. Un procédé selon la revendication 17 ou 18, dans lequel l'agent de liaison (L) est un composé de formule R1-A-R2, dans laquelle :
A désigne une chaîne aliphatique, aromatique ou une chaîne mixte aliphatique et aromatique, substituée ou non, saturée ou insaturée ;
R1 désigne une amine primaire ou un radical chimique porteur d'une amine primaire; et
R2 désigne un groupement fonctionnel capable de réagir avec un groupement carboxyl, thiol ou amine.

23. Un procédé selon la revendication 22, dans lequel l'agent de liaison (L) est un alkyl dihydrazide ou un diaminoalkyl.

24. Un procédé selon la revendication 19 ou 20, dans lequel l'espaceur S est un aminothiol et l'agent de liaison L'est un sucinimidyl maleimidyl alkyl.

25. Un procédé selon la revendication 19 ou 20, dans lequel l'espaceur S est un diaminoalkyl ou un dihydrazide, et l'agent de liaison L'est choisi parmi les composés dissucinimidyl alkyls ou succinimidyl maleimido alkyls de formule (XIV) R3-B-R4 dans laquelle B est un groupement alkyl, R3 est un groupement succinimidyl et R4 est un groupement succinimidyl ou maleimido.

26. Une composition pharmaceutique comprenant un conjugué selon la revendication 6 ou obtenu par le procédé selon l'une des revendications 15 à 25.

## Claims

1. A pneumococcus type 5 capsular polysaccharide which is aminated on the terminal aldehyde group, consisting of repeating units, at least 85% of the repeating units of which correspond to formula (II) 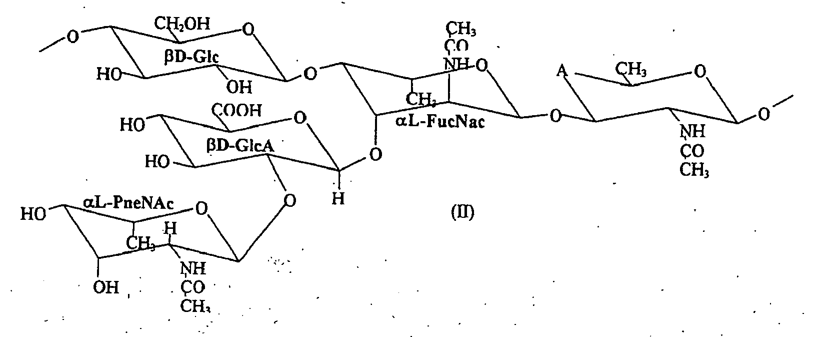 in which A is independently and randomly C=O or CHOH.

2. A polysaccharide according to Claim 1, in which at least 90% of the repeating units correspond to formula (II).

3. A polysaccharide according to Claim 2, in which at least 95% of the repeating units correspond to formula (II).

4. A polysaccharide according to one of Claims 1 to 3, in which at least 95% of the repeating units corresponding to formula (II) correspond to formula II" 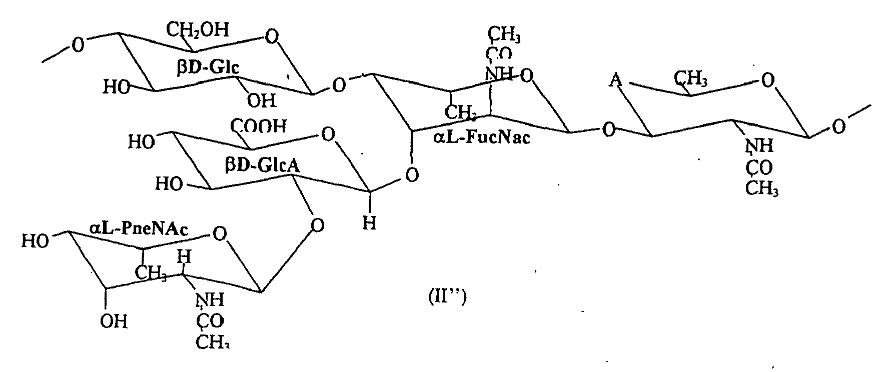 in which A is CHOH.

5. A polysaccharide according to one of Claims 1 to 3, in which 85 to 95% of the repeating units corresponding to formula (II) correspond to formula II' 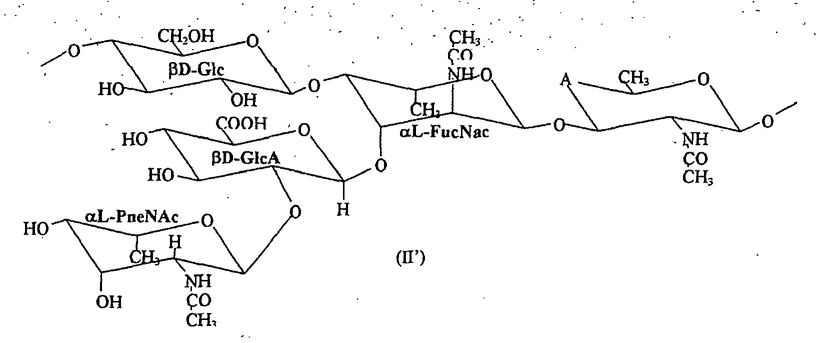 in which A is C=O.

6. A conjugate in which a polysaccharide according to one of Claims 1 to 5 is coupled to a carrier polypeptide (P).

7. A method for producing an aminated pneumococcus type 5 capsular polysaccharide, according to which the polysaccharide is subjected to a reductive amination in the presence of a reducing agent selective for a Schiff base, at a pH of 4 to 6.5, for a period not exceeding 4 hours.

8. A method according to Claim 7, in which the polysaccharide is subjected to a reductive amination at a pH of 5 to 6.

9. A method according to Claim 7 or 8, in which the polysaccharide is subjected to a reductive amination for a period not exceeding 2 hours.

10. A method according to Claim 7, .8 or 9, in which the reducing agent selective for a Schiff base is cyanoborohydride or the pyridine borane complex.

11. A method for producing an aminated pneumococcus type 5 capsular polysaccharide, according to which (i) the polysaccharide is reacted with an agent capable of reducing a ketone function, (ii) the reduced polysaccharide is fragmented, and (iii) the reduced and fragmented polysaccharide is subjected to a reductive amination.

12. A method according to Claim 11, in which the polysaccharide which is reacted with the agent capable of reducing a ketone function is in native form.

13. A method according to Claim 11 or 12, in which the agent capable of reducing a ketone function is NaBH₄.

14. A method according to Claim 11, 12 or 13, in which the reduced polysaccharide is fragmented by oxidative free-radical depolymerization.

15. A method for producing a conjugate of formula Ps-*CH₂-NH*-P, according to which a method according to one of Claims 7 to 10 is used, in which the pneumococcus type 5 capsular polysaccharide (Ps) is coupled, by reductive amination, with a carrier polypeptide '(P), in the presence of a reducing agent selective for a Schiff base, at a pH of 4 to 6.5, preferably of 5 to 6, for a period not exceeding 4 hours.

16. A method for producing a conjugate of formula Ps-*CH₂-NH*-P, according to which a method according to one of Claims 11 to 14 is used, in which the reduced and fragmented pneumococcus type 5 capsular polysaccharide (Ps) is coupled, be reductive amination, with a carrier polypeptide (P) .

17. A method for producing a conjugate of formula Ps-*CH₂-NH*-L-P, accordirig to which:
(i) the method according to one of Claims 7 to 10 is used, in which the pneumococcus type 5 capsular polysaccharide (Ps) is coupled, by reductive amination, to a linking agent (L) having at least one primary amine function, so as to form an aminated and activated polysaccharide of formula Ps-*CH₂-NH*-L, and the activated polysaccharide is coupled to a carrier polypeptide (P), in order to obtain the conjugate of formula Ps-*CH₂-NH*-L-P; or alternatively,
(ii) the method according to one of Claims 7 to 10 is used, in which the pneumococcus type 5 capsular polysaccharide (Ps) is coupled, by reductive amination, to an activated carrier polypeptide of formula L-P, in which L is a linking agent having at least one primary amine function, in order to obtain the conjugate of formula Ps-*CH₂-NH*-L-P.

18. A method for producing a conjugate of formula Ps-*CH₂-NH*-L-P, in which:
(i)
a) a method according to one of Claims 11 to 14 is used, in which the reduced and fragmented pneumococcus type 5 capsular polysaccharide (Ps) is coupled, by reductive amination, to a linking agent (L) having at least one primary amine function, so as to form an aminated and activated polysaccharide of formula Ps-*CH₂-NH*-L, and
b) the activated polysaccharide is coupled to a carrier polypeptide (P), in.order to obtain the conjugate of formula Ps-*CH₂-NH*-L-P; or alternatively,
(ii) the method according to one of Claims 11 to 14 is used, in which the reduced and fragmented pneumococcus type 5 capsular polysaccharide (Ps) is coupled, by reductive amination, to an activated carrier polypeptide of formula L-P, in which L is a linking agent having at least one primary amine function, in order to obtain the conjugate of formula Ps-*CH₂-NH*-L-P.

19. A method for producing a conjugate of formula Ps-*CH₂-NH*-S-L'-P, in which:
(i) a) the method according to one of Claims 7 to 10 is used, in which the pneumococcus type 5 capsular polysaccharide (Ps) is coupled, by reductive amination, to a spacer (S) having at least one primary amine function, so as to form an aminated and derivatized polysaccharide of formula Ps-*CH₂-NH*-S, and b) the derivatized polysaccharide is coupled with a linking agent (L'), in order to obtain an activated polysaccharide of formula Ps-*CH₂-NH*-S-L', then the activated polysaccharide is coupled with a carrier polypeptide (P), in order to obtain the conjugate of formula Ps-*CH₂*-*NH*-S-L'-P; or alternatively,
(ii) b) the derivatized polysaccharide is coupled with an activated carrier polypeptide of formula L'-P, in which L' is a linking agent, in order to obtain the conjugate of formula Ps-*CH₂-NH*-S-L'-P.

20. A method for producing a conjugate of formula Ps-*CH₂-NH-*S-L'-P, in which:
(i)
a) the method according to one of Claims 11 to 14 is used, in which the reduced and fragmented pneumococcus type 5 capsular polysaccharide (Ps) is coupled, by reductive amination, to a spacer (S) having at least one primary amine function, so as to form an aminated and derivatized polysaccharide of formula Ps-*CH₂-NH*-S, and
b) the derivatized polysaccharide is coupled with a linking agent (L'), in order to obtain an activated polysaccharide of formula Ps-*CH₂-NH*-S-L', then the activated polysaccharide is coupled with a carrier polypeptide (P), in order to obtain the conjugate of formula Ps-*CH₂-NH*-S-L'-P; or alternatively,
(ii) b) the derivatized polysaccharide is coupled with an activated carrier polypeptide of formula L'-P, in which L' is a linking agent, in order to obtain the conjugate of formula Ps-*CH₂-NH*-S-L'-P.

21. A method according to one of Claims 15 to 20, in which the carrier polypeptide P is diphtheria toxoid or tetanus toxoid.

22. A method according to Claim 17 or 18, in which the linking agent (L) is a compound of formula R1-A-R2, in which :
A denotes an aliphatic or aromatic chain or a mixed aliphatic and aromatic chain which may be substituted or unsubstituted, saturated or unsaturated;
R1 denotes a primary amine or a chemical radical carrying a primary amine; and
R2 denotes a functional group capable of reacting with a carbonyl, thiol or amine group.

23. A method according to Claim 22, in which the linking agent (L) is an alkyl dihydrazide or a diaminoalkyl.

24. A method according to Claim 19 or 20, in which the spacer S is an aminothiol and the linking agent L' is a succinimidylmaleimidylalkyl.

25. A method according to Claim 19 or. 20, in which the spacer S is a diaminoalkyl or a dihydrazide, and the linking agent L' is chosen from disuccinimidylalkyl or succinimidylmaleimidoalkyl compounds of formula (XIV) R3-B-R4 in which B is an alkyl group, R3 is a succinimidyl group and R4 is a succinimidyl or maleimido group.

26. A pharmaceutical composition comprising a conjugate according to Claim 6 or obtained using the method according to one of Claims 15 to 25.

## Patentansprüche

1. Kapsel-Polysaccharid von Pneumococcus Typ 5, aminiert im Bereich der terminalen Aldehydgruppe und bestehend aus wiederkehrenden Einheiten, von denen mindestens 85 % wiederkehrende Einheiten sind, die der Formel (II) 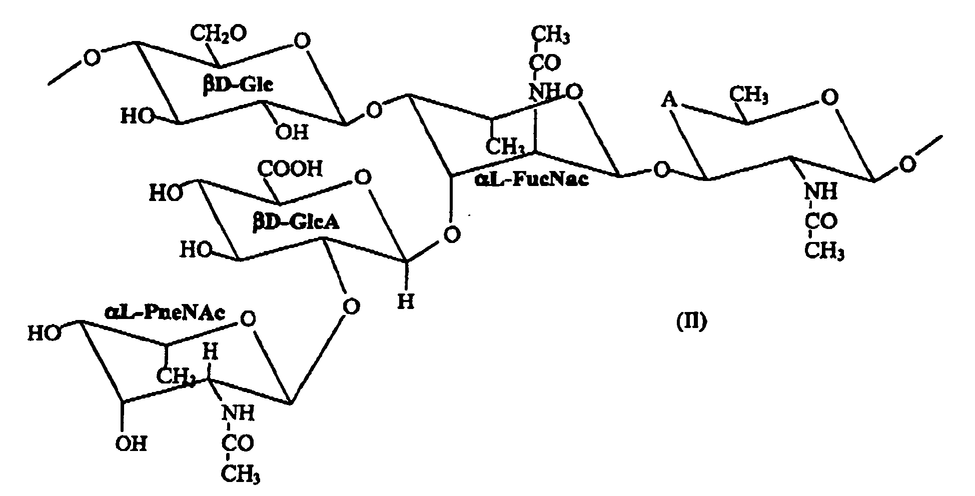 entsprechen, in der A in unabhängiger und zufälliger Weise C=O oder CHOH bedeutet.

2. Polysaccharid nach Anspruch 1, in dem mindestens 90 % der wiederkehrenden Einheiten der Formel (II) entsprechen.

3. Polysaccharid nach Anspruch 2, in dem mindestens 95 % der wiederkehrenden Einheiten der Formel (II) entsprechen.

4. Polysaccharid nach einem der Ansprüche 1 bis 3, in dem mindestens 95 % der wiederkehrenden Einheiten, die der Formel (II) entsprechen, der Formel II" 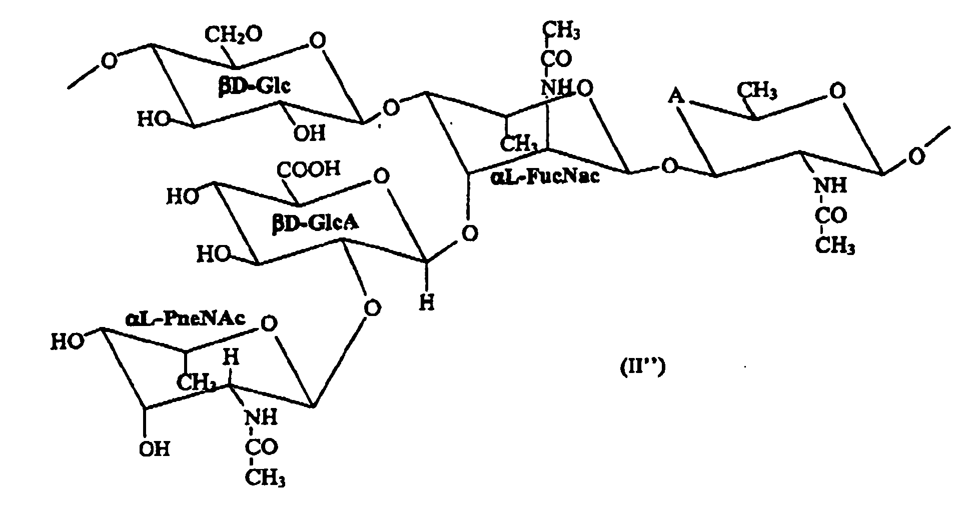 entsprechen, in der A CHOH ist.

5. Polysaccharid nach einem der Ansprüche 1 bis 3, in dem mindestens 85 bis 95 % der wiederkehrenden Einheiten, die der Formel (II) entsprechen, der Formel II' 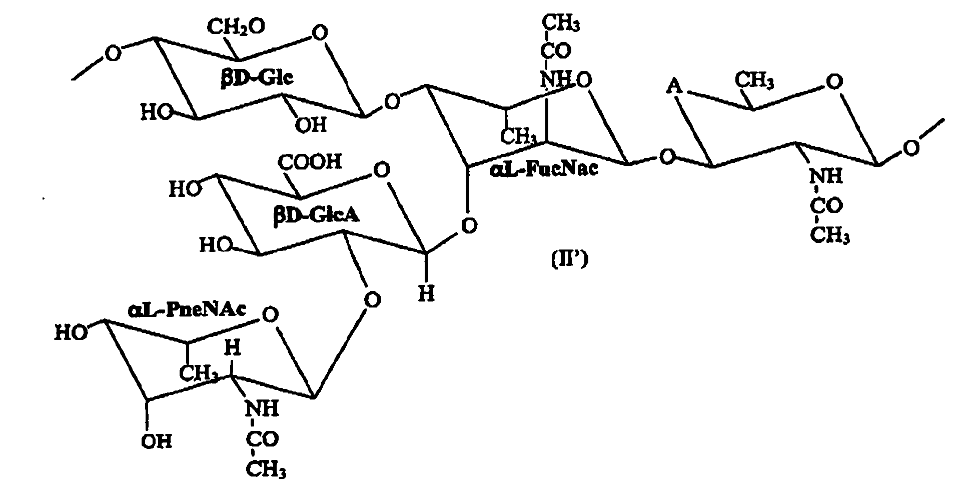 entsprechen, in der A C=O ist.

6. Konjugat, bei dem ein Polysaccharid nach einem der Ansprüche 1 bis 5 mit einem Träger-Polypeptid (P) gekoppelt ist.

7. Verfahren zur Herstellung eines aminierten Kapsel-Polysaccharides von Pneumococcus Typ 5, bei dem man das Polysaccharid einer reduktiven Aminierung in Anwesenheit eines selektiven Reduktionsmittels einer Schiff'schen Base bei einem pH-Wert von 4 bis 6,5 während einer Dauer unterzieht, die 4 Stunden nicht überschreitet.

8. Verfahren nach Anspruch 7, bei dem man das Polysaccharid einer reduktiven Aminierung bei einem pH-Wert von 5 bis 6 unterzieht.

9. Verfahren nach Anspruch 7 oder 8, bei dem man das Polysaccharid einer reduktiven Aminierung während einer Dauer unterzieht, die 2 Stunden nicht überschreitet.

10. Verfahren nach Anspruch 7, 8 oder 9, bei dem das selektive Reduktionsmittel einer Schiff'schen Base Cyanoborhydrid oder der Komplex Pyridin-Boran ist.

11. Verfahren zur Herstellung eines aminierten Kapsel-Polysaccharides von Pneumococcus Typ 5, bei dem man (i) das Polysaccharid mit einem Mittel zur Reaktion bringt, das geeignet ist, eine Ketonfunktion zu reduzieren, (ii) das reduzierte Polysaccharid fragmentiert und (iii) das reduzierte und fragmentierte Polysaccharid einer reduktiven Aminierung unterzieht.

12. Verfahren nach Anspruch 11, bei dem das Polysaccharid, das man mit dem Mittel zur Reaktion bringt, das geeignet ist, eine Ketonfunktion zu reduzieren, in natürlicher Form vorliegt.

13. Verfahren nach Anspruch 11 oder 12, bei dem das Mittel, das geeignet ist, eine Ketonfunktion zu reduzieren, NaBH₄ ist.

14. Verfahren nach Anspruch 11, 12 oder 13, bei dem man das reduzierte Polysaccharid durch oxidative radikalische Depolymerisation fragmentiert.

15. Verfahren zur Herstellung eines Konjugates der Formel Ps-*CH2-NH*-P, gemäß dem man ein Verfahren nach einem der Ansprüche 7 bis 10 durchführt, bei dem das Kapsel-Polysaccharid von Pneumococcus Typ 5 (Ps) durch reduktive Aminierung mit einem Träger-Polypeptid (P) in Anwesenheit eines selektiven Reduktionsmittels einer Schiff'schen Base bei einem pH-Wert von 4 bis 6,5, bevorzugt von 5 bis 6, während einer Dauer gekoppelt wird, die 4 Stunden nicht überschreitet.

16. Verfahren zur Herstellung eines Konjugates der Formel Ps-*CH2-NH*-P, gemäß dem man ein Verfahren nach einem der Ansprüche 11 bis 14 durchführt, bei dem das reduzierte und fragmentierte Kapsel-Polysaccharid von Pneumococcus Typ 5 (Ps) durch reduktive Aminierung mit einem Träger-Polypeptid (P) gekoppelt wird.

17. Verfahren zur Herstellung eines Konjugates der Formel Ps*-CH2-NH*-L-P, gemäß dem man:
(i) das Verfahren nach einem der Ansprüche 7 bis 10 durchführt, bei dem das Kapsel-Polysaccharid von Pneumococcus Typ 5 (Ps) durch reduktive Aminierung mit einem Bindungsmittel (L) gekoppelt wird, das mindestens eine primäre Aminfunktion besitzt, um ein aminiertes und aktiviertes Polysaccharid der Formel Ps-*CH2-NH*-L zu bilden, und man das aktivierte Polysaccharid mit einem Träger-Polypeptid (P) koppelt, um das Konjugat der Formel Ps-*CH2-NH*-L-P zu erhalten;
oder alternativ
(ii) das Verfahren nach einem der Ansprüche 7 bis 10 durchführt, bei dem das Kapsel-Polysaccharid von Pneumococcus Typ 5 (Ps) durch reduktive Aminierung mit einem aktivierten Träger-Polypeptid der Formel L-P gekoppelt wird, in der L ein Bindungsmittel ist, das mindestens eine primäre Aminfunktion besitzt, um das Konjugat der Formel Ps-*CH2-NH-*L-P zu erhalten.

18. Verfahren zur Herstellung eines Konjugates der Formel Ps-*CH2-NH*-L-P, gemäß dem man:
(i)
a) das Verfahren nach einem der Ansprüche 11 bis 14 durchführt, bei dem das reduzierte und fragmentierte Kapsel-Polysaccharid von Pneumococcus Typ 5 (Ps) durch reduktive Aminierung mit einem Bindungsmittel (L) gekoppelt wird, das mindestens eine primäre Aminfunktion besitzt, um ein aminiertes und aktiviertes Polysaccharid der Formel Ps-*CH2-NH*-L zu bilden, und
b) man das aktivierte Polysaccharid mit einem Träger-Polypeptid (P) koppelt, um das Konjugat der Formel Ps-*CH2*-*NH*-L-P zu erhalten;
oder alternativ
(ii) das Verfahren nach einem der Ansprüche 11 bis 14 durchführt, bei dem das reduzierte und fragmentierte Kapsel-Polysaccharid von Pneumococcus Typ 5 (Ps) durch reduktive Aminierung mit einem aktivierten Träger-Polypeptid der Formel L-P gekoppelt wird, in der L ein Bindungsmittel ist, das mindestens eine primäre Aminfunktion besitzt, um das Konjugat der Formel Ps-*CH2*-*NH*-L-P zu erhalten.

19. Verfahren zur Herstellung eines Konjugates der Formel Ps-*CH2*-*NH*-S-L'-P, gemäß dem man:
(i)
a) das Verfahren nach einem der Ansprüche 7 bis 10 durchführt, bei dem das Kapsel-Polysaccharid von Pneumococcus Typ 5 (Ps) durch reduktive Aminierung mit einem Zwischenstück (S) gekoppelt wird, die mindestens eine primäre Aminfunktion besitzt, um ein aminiertes und derivatisiertes Polysaccharid der Formel Ps-*CH2-NH*-S zu bilden, und
b) man das derivatisierte Polysaccharid mit einem Bindungsmittel (L') koppelt, um das aktivierte Polysaccharid der Formel Ps-*CH2NH*-S-L' zu erhalten, und man anschließend das aktivierte Polysaccharid mit einem Träger-Polypeptid (P) koppelt, um das Konjugat der Formel Ps-*CH2-NH*-S-L'-P zu erhalten; oder alternativ
(ii) b) das derivatisierte Polysaccharid mit einem aktivierten Träger-Polypeptid der Formel L'-P koppelt, in der L' ein Bindungsmittel ist, um das Konjugat der Formel Ps-*CH2-NH*-S-L'-P zu erhalten.

20. Verfahren zur Herstellung eines Konjugates der Formel Ps-*CH2-NH*-S-L'-P, gemäß dem man:
(i)
a) das Verfahren nach einem der Ansprüche 11 bis 14 durchführt, bei dem das reduzierte und fragmentierte Kapsel-Polysaccharid von Pneumococcus Typ 5 (Ps) durch reduktive Aminierung mit einem Zwischenstück (S) gekoppelt wird, die mindestens eine primäre Aminfunktion besitzt, um ein aminiertes und derivatisiertes Polysaccharid der Formel Ps-*CH2-NH*-S zu bilden, und
b) man das derivatisierte Polysaccharid mit einem Bindungsmittel (L') koppelt, um ein aktiviertes Polysaccharid der Formel Ps-*CH2NH*-S-L' zu erhalten, und man anschließend das aktivierte Polysaccharid mit einem Träger-Polypeptid (P) koppelt, um das Konjugat der Formel Ps-*CH2-NH*-S-L'-P zu erhalten;
oder alternativ
(ii) b) das derivatisierte Polysaccharid mit einem aktivierten Träger-Polypeptid der Formel L'-P koppelt, in der L' ein Bindungsmittel ist, um das Konjugat der Formel Ps-*CH2-NH-*S-L'-P zu erhalten.

21. Verfahren nach einem der Ansprüche 15 bis 20, bei dem das Träger-Polypeptid P das Anatoxin von Diphterie oder Tetanus ist.

22. Verfahren nach Anspruch 17 oder 18, bei dem das Bindungsmittel L eine Verbindung der Formel R1-A-R2 ist, in der:
A eine aliphatische, aromatische oder eine gemischte aliphatische und aromatische, substituierte oder nicht substituierte, gesättigte oder ungesättigte Kette bezeichnet;
R1 ein primäres Amin oder einen chemischen Rest bezeichnet, der Träger eines primären Amins ist; und
R2 eine funktionelle Gruppe bezeichnet, die geeignet ist, mit einer Gruppe Carboxyl, Thiol oder Amin zu reagieren.

23. Verfahren nach Anspruch 22, bei dem das Bindungsmittel (L) ein Alkyl-dihydrazid oder ein Diaminoalkyl ist.

24. Verfahren nach Anspruch 19 oder 20, bei dem das Zwischenstück (S) ein Aminothiol ist und das Bindungsmittel (L) ein Succinimidyl-maleimidyl-alkyl ist.

25. Verfahren nach Anspruch 19 oder 20, bei dem die Zwischenverbindung (S) ein Diaminoalkyl oder ein Dihydrazid ist und das Bindungsmittel (L') ausgewählt wird unter den Verbindungen Disuccinimidyl-alkyl oder Succinimidyl-maleimidoalkyl der Formel (XIV)
R3-B-R4, in der B eine Gruppe Alkyl ist, R3 eine Gruppe Succinimidyl ist und R4 eine Gruppe Succinimidyl oder Maleimido ist.

26. Pharmazeutische Zusammensetzung, umfassend ein Konjugat nach Anspruch 6 oder wie es durch das Verfahren nach einem der Ansprüche 15 bis 25 erhalten wird.
